# EUROPEAN PATENT SPECIFICATION

(11) **EP 3 131 889 B1**
(45) Date of publication and mention of the grant of the patent: **06.06.2018**
(21) Application number: 15718309.6
(22) Date of filing: 06.04.2015
(51) Int. Cl.: C07D 333/16, C07D 333/18, C07C 321/08, C11B 9/00, A23L 27/20

(54) **NOVEL ORGANOLEPTIC COMPOUNDS AND THEIR USE IN FLAVOR AND FRAGRANCE COMPOSITIONS**
NEUARTIGE ORGANOLEPTISCHE VERBINDUNGEN UND IHRE VERWENDUNG IN GESCHMACKS- UND DUFTSTOFFZUSAMMENSETZUNGEN
NOUVEAUX COMPOSÉS ORGANOLEPTIQUES ET LEUR UTILISATION DANS DES COMPOSITIONS D'ARÔME ET DE FRAGRANCE

(30) Priority: 10.04.2014 US 201461977702 P
(43) Date of publication of application: 22.02.2017
(73) Proprietor: International Flavors & Fragrances Inc., New York, NY 10019 (US)
(72) Inventor: AGYEMANG, David O., Old Bridge, NJ 08857 (US); BRAIN, Anja Van Kippersluis, Colts Neck, New Jersey 07722 (US); CAI, Tingwei, Holmdel, New Jersey 07733 (US); CANNON, Robert J., Fair Haven, New Jersey 07704 (US); CHEN, Michael Zhen, Aberdeen, New Jersey 07747 (US); CURTO, Nicole L., Little Falls, New Jersey 07424 (US); JANCZUK, Adam Jan, Old Bridge, New Jersey 08857 (US); JONES, Paul D., Aberdeen, New Jersey 07747 (US); KAZIMIERSKI, Arkadiusz, Old Bridge, New Jersey 08857 (US); LI, Jing, Aberdeen, New Jersey 07747 (US); RODRIGUEZ, David, Belleville, New Jersey 07109 (US)
(74) Representative: Barker Brettell LLP
(86) International application number: PCT/US2015/024470
(87) International publication number: WO 2015/157153

(56) References cited:
- CH-A- 556 145
- US-A1- 2002 037 349
- CANNON, ROBERT J. ET AL: "Identification, Synthesis, and Characterization of Novel Sulfur-Containing Volatile Compounds from the In-Depth Analysis of Lisbon Lemon Peels (Citrus limon L. Burm. f. cv. Lisbon)", JOURNAL OF AGRICULTURAL AND FOOD CHEMISTRY, vol. 63, no. 7, 31 January 2015 (2015-01-31), pages 1915-1931, XP002739823, ISSN: 0021-8561, DOI: 10.1021/JF505177R
- GUENTERT M ET AL: "THERMALLY DEGRADED THIAMIN A POTENT SOURCE OF INTERESTING FLAVOR COMPOUNDS", WATER-SOLUBLE POLYMERS: SYNTHESIS, SOLUTION PROPERTIES AND APPLICATIONS, AMERICAN CHEMICAL SOCIETY, WASHINGTON, DC, US, vol. 490, 1 January 1992 (1992-01-01), pages 140-163, XP000864382, ISBN: 978-0-541-23408-9

## Description

### Field of the Invention

The present invention relates to new chemical entities and their use as flavor and fragrance materials.

### Background of the Invention

There is an ongoing need for flavor chemicals that enhance or provide new flavors for food preparations. There is a similar need in the fragrance industry to provide new chemicals to give perfumers and other persons the ability to create new fragrances for perfumes, colognes and personal care products. Those with skill in the art appreciate how differences in the chemical structures of the molecules can result in significant differences in the odor, notes and characteristics. The identification of structural variations and discovery of new chemicals enable the creation of new flavors and fragrances.

"Thermally degraded thiamin: a potent source of interesting flavor compounds" (Guentert M et al., 1992) discusses obtaining flavor compounds by heating aqueous solutions of thiamin hydrochloride with different concentrations and differently adjusted pH values in an autoclave for various times, and applying the simultaneous distillation/extraction method according to Likens-Nickerson. The flavor concentrate was pre-separated and the different fractions were subsequently analyzed. Various unknown compounds were isolated in microgram-quantities to elucidate their structures by IR, NMR, and mass spectrometry, and to check their olfactory properties.
The identified compounds were used to explain the various degradation pathways of thermally treated thiamin. Their occurrence, formation, sensory impression, and spectroscopic data are discussed.

US 2002/037349 discloses flavor precursors having the formula R1-S-CO-O-R2, wherein R1 is a heterocyclic radical selected from the group consisting of: 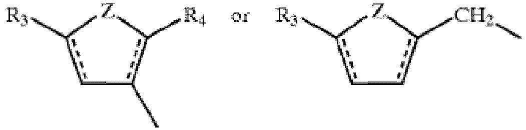 wherein Z is an oxygen or a sulfur atom, R3 and R4 represent hydrogen or an C1-C4 alkyl group and the symbol represents a single or double bond, and R2 is derived from a group of primary alcohol compounds consisting of C1-C18 alkanols, glycerol and mono-, oligo- and polysaccharides, wherein the oxygen of the R2-O- moiety is attached to a primary carbon atom of R2 '349 further discloses the foodstuffs provided with a flavor precursor specified above.

CH 556145 discloses use of at least one sulfur derivative of the formula below as a perfuming ingredient for the preparation of perfumes and scented products and/or flavorings for preparation of artificial flavors and food/drink flavoring: 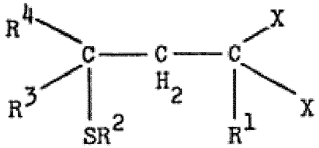 wherein one X represents a hydroxyl group or an acyloxy group, the other X represents a hydrogen atom; and wherein R1, R2, R3 and R4 may be the same or different, and each represents a hydrogen atom or a univalent aliphatic hydro-carbyl group; or R1 and R3 or R4, together with the intervening carbon atoms, constitute a cyclo-aliphatic ring

### Summary of the Invention

The present invention is directed to the use of novel chemicals to enhance the flavor of foodstuff, chewing gums, dental and oral hygiene products and medicinal products. In addition, the present invention provides novel chemicals, and their use to enhance the fragrance of perfumes, toilet waters, colognes, personal products and the like.

More specifically, the present invention is directed to novel thiophen-2-yl compounds and a method of improving, enhancing or modifying a flavor or a fragrance composition through the addition of an olfactory acceptable amount of thiophen-2-yl compounds represented by Formula I set forth below: 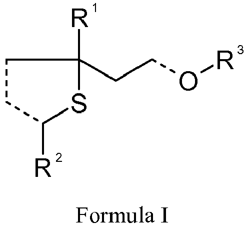 wherein
R¹ is a C₁-C₄ alkyl group;
R² is selected from the group consisting of hydrogen, a C₁-C₄ alkyl group optionally containing a hydroxyl group and a C₁-C₄ alkenyl group optionally containing a hydroxyl group;
R³ is selected from the group consisting of hydrogen, a C₁-C₄ alkyl group and -C(O)R⁴, wherein R⁴ is selected from the group consisting of hydrogen and a C₁-C₄ alkyl group;
one of the dotted lines in the ring represents an optional carbon-carbon double bond; and the dotted line in the side chain represents an optional carbon-oxygen double bond,
with the proviso that, when the optional carbon-oxygen double bond is present, R³ is absent.

Another embodiment of the present invention relates to novel thiophen-2-yl compounds represented by Formula II set forth below: 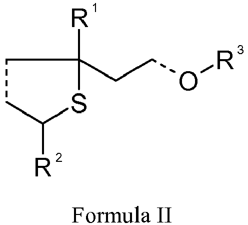
wherein R¹, R² and R³ are defined as above;
the dotted line in the ring represents an optional carbon-carbon double bond; and the dotted line in the side chain represents an optional carbon-oxygen double bond,
with the proviso that, when the optional carbon-oxygen double bond is present, R³ is absent.

Another embodiment of the present invention relates to novel thiophen-2-yl compounds represented by Formula III set forth below: 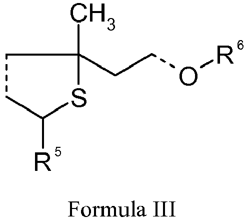 wherein
R⁵ represents hydrogen, isopropyl or 1-hydroxy-1-methyl-ethyl;
R⁶ represents hydrogen;
the dotted line in the ring represents an optional carbon-carbon double bond; and the dotted line in the side chain represents an optional carbon-oxygen double bond,
with the proviso that, when the optional carbon-oxygen double bond is present, R⁶ is absent; and when R⁵ represents an isopropyl group and the optional carbon-carbon double bond is absent, the optional carbon-oxygen double bond is present and R⁶ is absent.

Another embodiment of the present invention relates to novel thiophen-2-yl compounds represented by Formula IV set forth below: 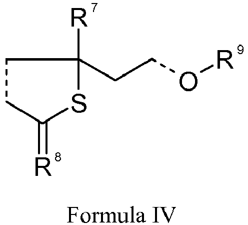 wherein
R⁷ is a C₁-C₄ alkyl group;
R⁸ represents a C₁-C₄ alkyl group optionally containing a hydroxyl group;
R⁹ is selected from the group consisting of hydrogen, a C₁-C₄ alkyl group and -C(O)R¹⁰, wherein R¹⁰ is selected from the group consisting of hydrogen and a C₁-C₄ alkyl group;
the dotted line in the ring represents an optional carbon-carbon double bond; and the dotted line in the side chain represents an optional carbon-oxygen double bond,
with the proviso that, when the optional carbon-oxygen double bond is present, R⁹ is absent.

Another embodiment of the present invention relates to novel thiophen-2-yl compounds represented by Formula V set forth below: 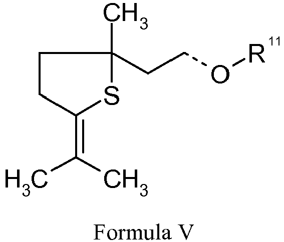
wherein R¹¹ is selected from the group consisting of hydrogen and a C₁-C₄ alkyl group; and the dotted line represents an optional carbon-oxygen double bond,
with the proviso that, when the optional carbon-oxygen double bond is present, R¹¹ is absent.

Another embodiment of the present invention relates to novel thiophen-2-yl compounds represented by Formula VI set forth below: 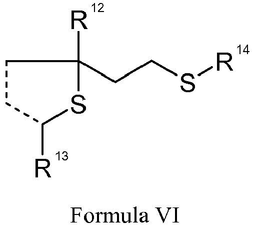 wherein
R¹² is a C₁-C₄ alkyl group;
R¹³ is selected from the group consisting of hydrogen, a C₁-C₄ alkyl group optionally containing a hydroxyl group and a C₁-C₄ alkenyl group optionally containing a hydroxyl group;
R¹⁴ is selected from the group consisting of hydrogen, a C₁-C₄ alkyl group and - C(O)R¹⁵, wherein R¹⁵ is selected from the group consisting of hydrogen and a C₁-C₄ alkyl group; and
one of the dotted lines represents an optional carbon-carbon double bond.

Another embodiment of the present invention relates to novel thiophen-2-yl compounds represented by Formula VII set forth below: 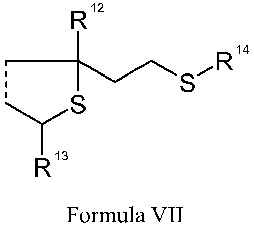
wherein R¹², R¹³ and R¹⁴ are defined as above; and
the dotted line represents an optional carbon-carbon double bond.

Another embodiment of the present invention relates to novel thiophen-2-yl compounds represented by Formula VIII set forth below: 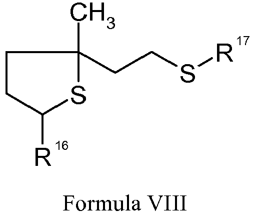
wherein R¹⁶ represents hydrogen, isopropyl or 1-hydroxy-1-methyl-ethyl; and
R¹⁷ is selected from the group consisting of a C₁-C₄ alkyl group and -C(O)R¹⁸, wherein R¹⁸ is selected from the group consisting of hydrogen and a C₁-C₄ alkyl group.

Another embodiment of the present invention relates to novel oct-6-enyl compounds represented by Formula IX set forth below: 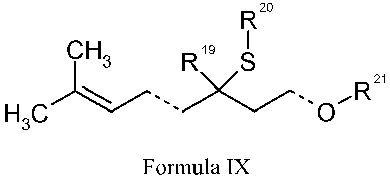 wherein
R¹⁹ is a C₁-C₄ alkyl group;
R²⁰ is selected from the group consisting of hydrogen and -C(O)R²², wherein R²² is selected from the group consisting of hydrogen and a C₁-C₄ alkyl group;
R²¹ is selected from the group consisting of hydrogen, a C₁-C₄ alkyl group and - C(O)R²³, wherein R²³ is selected from the group consisting of hydrogen and a C₁-C₄ alkyl group;
the dotted lines represent an optional carbon-carbon double bond and an optional carbon-oxygen double bond,
with the proviso that, when the optional carbon-oxygen double bond is present, R²¹ is absent.

Another embodiment of the present invention relates to novel oct-6-enyl compounds represented by Formula X set forth below: 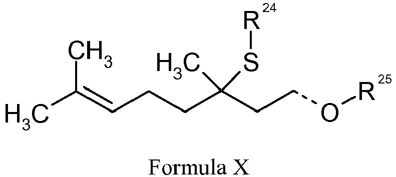 wherein Formula X represents an oct-6-enyl compound selected from the group consisting of:
3-mercapto-3,7-dimethyl-oct-6-en-1-ol;
3-mercapto-3,7-dimethyl-oct-6-enal;
S-[1-(2-hydroxy-ethyl)-1,5-dimethyl-hex-4-enyl]-thioacetate; and
3-acetylsulfanyl-3,7-dimethyl-oct-6-enyl acetate.

Another embodiment of the present invention relates to novel oct-6-enyl compounds represented by Formula XI set forth below: 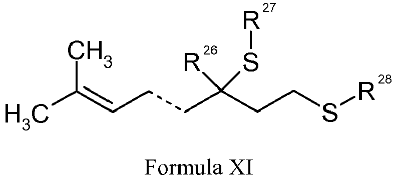 wherein
R²⁶ is a C₁-C₄ alkyl group;
R²⁷ is selected from the group consisting of hydrogen, a C₁-C₄ alkyl group and - C(O)R²⁹, wherein R²⁹ is selected from the group consisting of hydrogen and a C₁-C₄ alkyl group;
R²⁸ is selected from the group consisting of hydrogen, a C₁-C₄ alkyl group and - C(O)R³⁰, wherein R³⁰ is selected from the group consisting of hydrogen and a C₁-C₄ alkyl group; and
the dotted line represents an optional carbon-carbon double bond.

Another embodiment of the present invention relates to novel oct-6-enyl compounds represented by Formula XII set forth below: 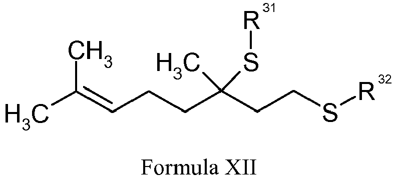 wherein R³¹ and R³² are each independently selected from the group consisting of hydrogen, a C₁-C₄ alkyl group and -C(O)CH₃.

Another embodiment of the present invention relates to novel thietane compounds represented by Formula XIII set forth below: 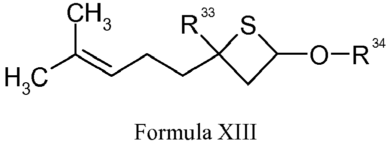 wherein R³³ and R³⁴ are each independently selected from the group consisting of hydrogen and a C₁-C₄ alkyl group.

Another embodiment of the present invention relates to novel thietane compounds represented by Formula XIV set forth below: 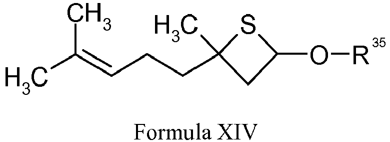 wherein R³⁵ is selected from the group consisting of hydrogen and a methyl group.

Another embodiment of the invention is directed to a flavor composition comprising the novel compounds provided above and a material selected from the group consisting of a foodstuff, a chewing gum, a dental product, an oral hygiene product and a medicinal product.

Another embodiment of the invention is directed to a process of augmenting, enhancing or imparting taste to a material by incorporating an olfactory acceptable amount of the novel compounds provided above.

Another embodiment of the present invention relates to a fragrance composition comprising the novel compounds provided above.

Another embodiment of the invention is directed to a method of improving, enhancing or modifying a fragrance composition by incorporating an olfactory acceptable amount of the novel compounds provided above.

These and other embodiments of the present invention will be apparent by reading the following specification.

### Detailed Description of the Invention

The novel compounds of the present invention are illustrated, for example, by following examples.
2-(2-Methyl-tetrahydro-thiophen-2-yl)-ethanol: 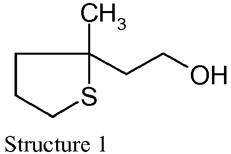
2-(2-Methyl-2,5-dihydro-thiophen-2-yl)-ethanol: 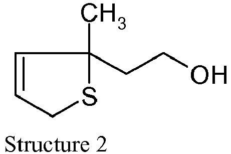
2-(2-Methyl-2,3-dihydro-thiophen-2-yl)-ethanol: 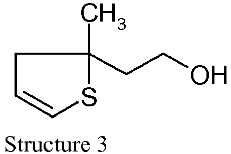
(2-Methyl-tetrahydro-thiophen-2-yl)-acetaldehyde: 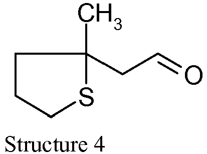
(2-Methyl-2,5-dihydro-thiophen-2-yl)-acetaldehyde: 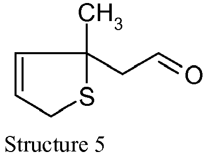
(2-Methyl-2,3-dihydro-thiophen-2-yl)-acetaldehyde: 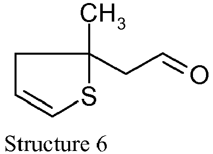
2-(2-Methyl-tetrahydro-thiophen-2-yl)-ethyl acetate: 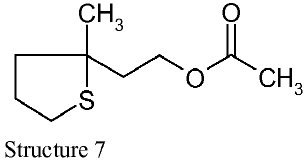
2-(2-Methyl-2,5-dihydro-thiophen-2-yl)-ethyl acetate: 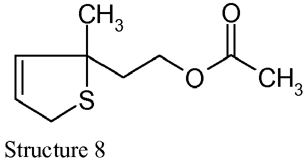
2-(2-Methyl-2,3-dihydro-thiophen-2-yl)-ethyl acetate: 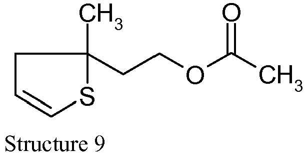
2-(5-Isopropyl-2-methyl-tetrahydro-thiophen-2-yl)-ethanol: 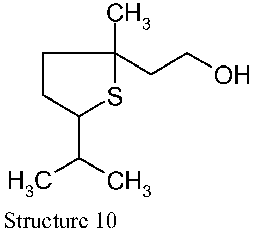
2-(5-Isopropyl-2-methyl-2,5-dihydro-thiophen-2-yl)-ethanol: 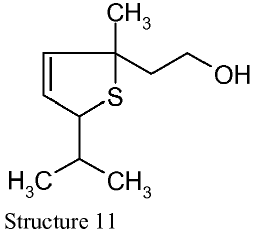
2-(5-Isopropyl-2-methyl-2,3-dihydro-thiophen-2-yl)-ethanol: 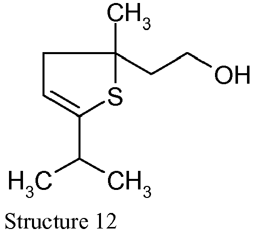
(5-Isopropyl-2-methyl-tetrahydro-thiophen-2-yl)-acetaldehyde: 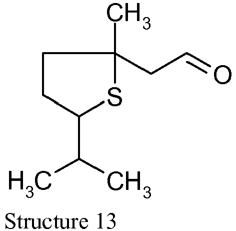
(5-Isopropyl-2-methyl-2,5-dihydro-thiophen-2-yl)-acetaldehyde: 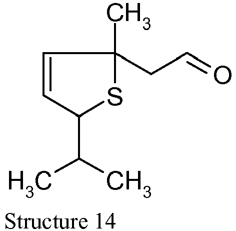
(5-Isopropyl-2-methyl-2,3-dihydro-thiophen-2-yl)-acetaldehyde: 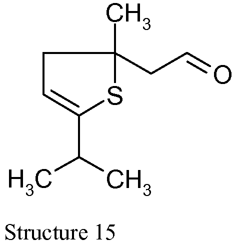
2-(5-Isopropyl-2-methyl-tetrahydro-thiophen-2-yl)-ethyl acetate: 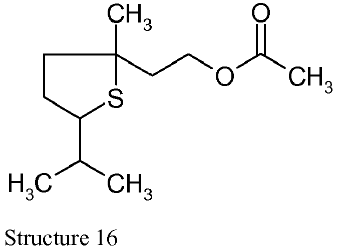
2-(5-Isopropyl-2-methyl-2,5-dihydro-thiophen-2-yl)-ethyl acetate: 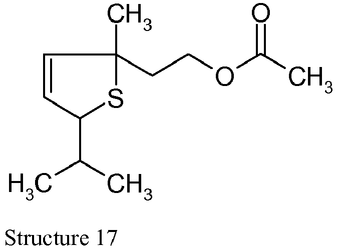
2-(5-Isopropyl-2-methyl-2,3-dihydro-thiophen-2-yl)-ethyl acetate : 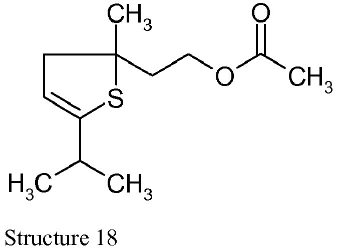
2-(5-Isopropenyl-2-methyl-tetrahydro-thiophen-2-yl)-ethanol: 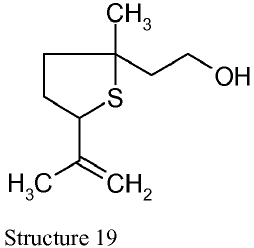
2-(5-Isopropenyl-2-methyl-2,5-dihydro-thiophen-2-yl)-ethanol: 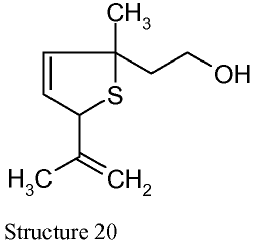
2-(5-Isopropenyl-2-methyl-2,3-dihydro-thiophen-2-yl)-ethanol: 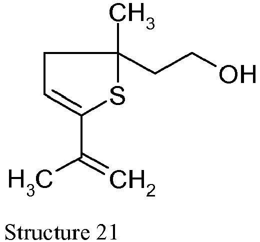
(5-Isopropenyl-2-methyl-tetrahydro-thiophen-2-yl)-acetaldehyde: 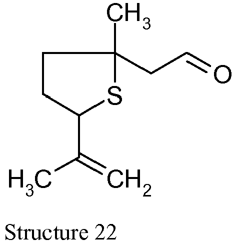
(5-Isopropenyl-2-methyl-2,5-dihydro-thiophen-2-yl)-acetaldehyde: 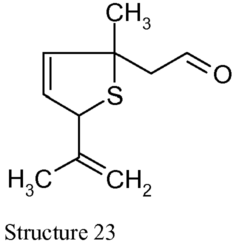
(5-Isopropenyl-2-methyl-2,3-dihydro-thiophen-2-yl)-acetaldehyde: 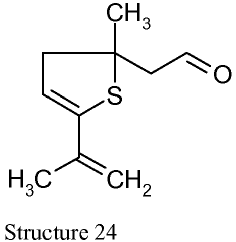
2-(5-Isopropenyl-2-methyl-tetrahydro-thiophen-2-yl)-ethyl acetate: 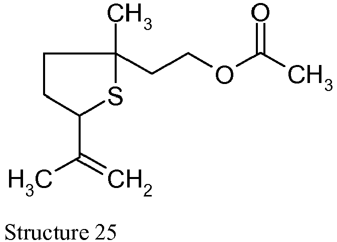
2-(5-Isopropenyl-2-methyl-2,5-dihydro-thiophen-2-yl)-ethyl acetate: 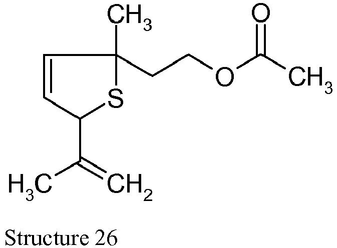
2-(5-Isopropenyl-2-methyl-2,3-dihydro-thiophen-2-yl)-ethyl acetate: 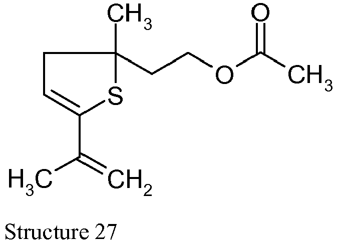
2-(5-Isopropylidene-2-methyl-tetrahydro-thiophen-2-yl)-ethanol: 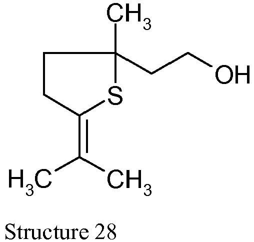
2-(5-Isopropylidene-2-methyl-2,5-dihydro-thiophen-2-yl)-ethanol: 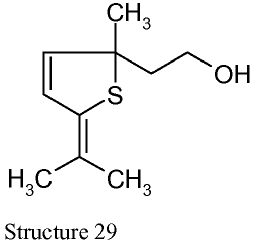
(5-Isopropenyl-2-methyl-tetrahydro-thiophen-2-yl)-acetaldehyde: 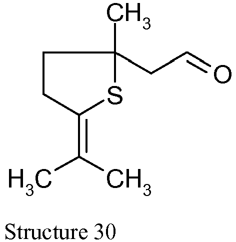
(5-Isopropylidene-2-methyl-2,5-dihydro-thiophen-2-yl)-acetaldehyde: 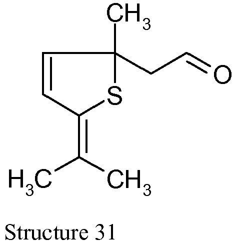
2-(5-Isopropenyl-2-methyl-tetrahydro-thiophen-2-yl)-ethyl acetate: 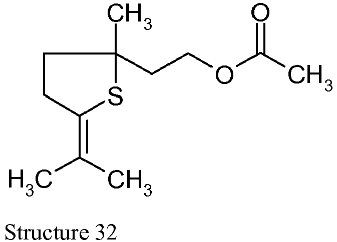
2-(5-Isopropylidene-2-methyl-2,5-dihydro-thiophen-2-yl)-ethyl acetate: 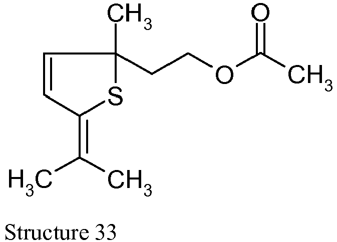
[5-(1-Hydroxy-1-methyl-ethyl)-2-methyl-tetrahydro-thiophen-2-yl] -acetaldehyde: 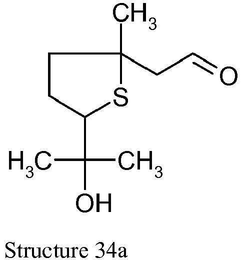
2-[5-(1-Hydroxy-1-methyl-ethyl)-2-methyl-tetrahydro-thiophen-2-yl] -ethyl acetate: 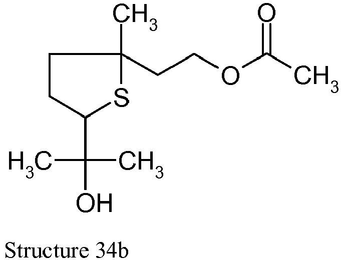
2-[5-(1-Hydroxy-1-methyl-ethyl)-2-methyl-2,5-dihydro-thiophen-2-yl]-ethyl acetate: 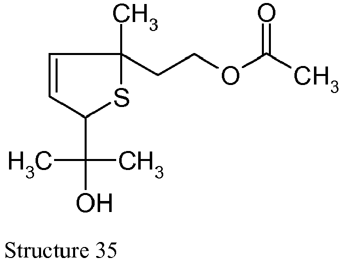
2-[5-(1-Hydroxy-1-methyl-ethyl)-2-methyl-2,3-dihydro-thiophen-2-yl]-ethyl acetate: 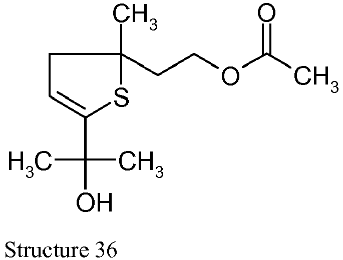
2-(2-Methyl-tetrahydro-thiophen-2-yl)-ethanethiol: 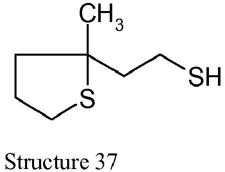
2-(2-Methyl-2,5-dihydro-thiophen-2-yl)-ethanethiol: 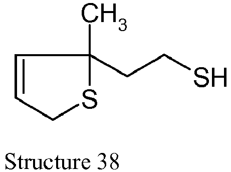
2-(2-Methyl-2,3-dihydro-thiophen-2-yl)-ethanethiol: 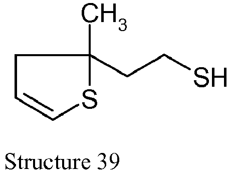
2-Methyl-2-(2-methylsulfanyl-ethyl)-tetrahydro-thiophene: 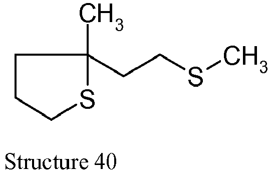
2-Methyl-2-(2-methylsulfanyl-ethyl)-2,5-dihydro-thiophene: 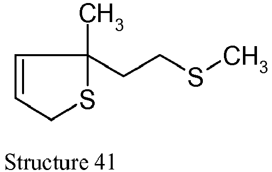
2-Methyl-2-(2-methylsulfanyl-ethyl)-2,3-dihydro-thiophene: 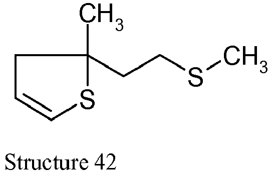
2-(5-Isopropyl-2-methyl-tetrahydro-thiophen-2-yl)-ethanethiol: 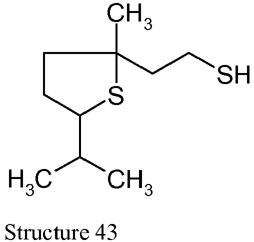
2-(5-Isopropyl-2-methyl-2,5-dihydro-thiophen-2-yl)-ethanethiol: 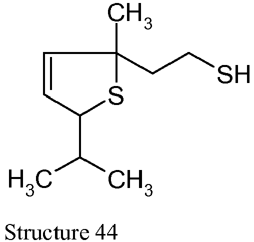
2-(5-Isopropyl-2-methyl-2,3-dihydro-thiophen-2-yl)-ethanethiol: 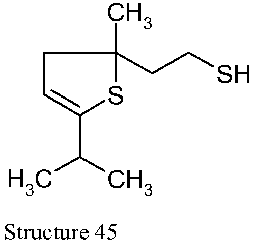
5-Isopropyl-2-methyl-2-(2-methylsulfanyl-ethyl)-tetrahydro-thiophene: 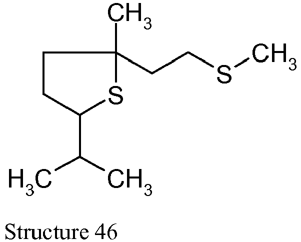
5-Isopropyl-2-methyl-2-(2-methylsulfanyl-ethyl)-2,5-dihydro-thiophene: 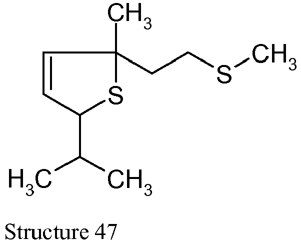
5-Isopropyl-2-methyl-2-(2-methylsulfanyl-ethyl)-2,3-dihydro-thiophene: 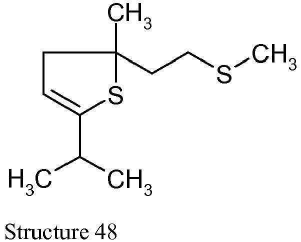
S-[2-(2-Methyl-tetrahydro-thiophen-2-yl)-ethyl]-thioacetate: 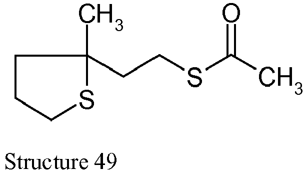
S-[2-(2-Methyl-2,5-dihydro-thiophen-2-yl)-ethyl]-thioacetate: 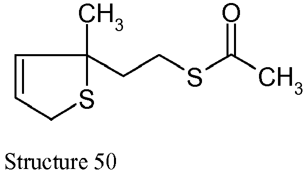
S-[2-(2-Methyl-2,3-dihydro-thiophen-2-yl)-ethyl]-thioacetate: 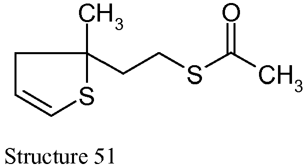
S-[2-(5-Isopropyl-2-methyl-tetrahydro-thiophen-2-yl)-ethyl]-thioacetate: 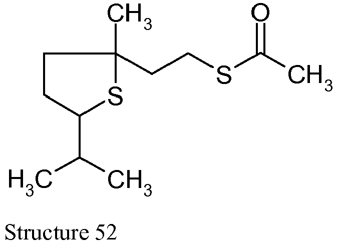
S-[2-(5-Isopropyl-2-methyl-2,5-dihydro-thiophen-2-yl)-ethyl]-thioacetate: 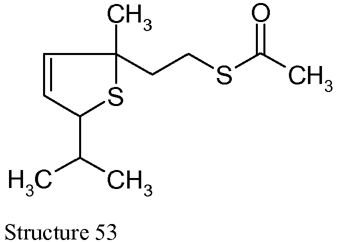
S-[2-(5-Isopropyl-2-methyl-2,3-dihydro-thiophen-2-yl)-ethyl]-thioacetate: 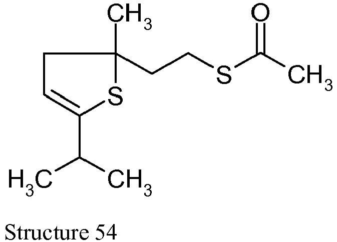
3-Mercapto-3,7-dimethyl-oct-6-en-1-ol: 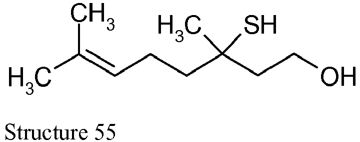
3-Mercapto-3,7-dimethyl-octa-4,6-dien-1-ol: 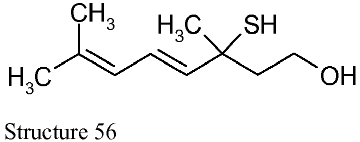
3-Mercapto-3,7-dimethyl-oct-6-enal: 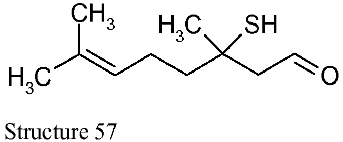
3-Mercapto-3,7-dimethyl-octa-4,6-dienal: 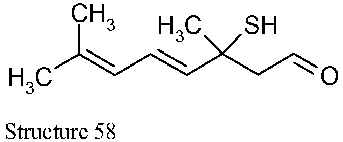
3-Mercapto-3,7-dimethyl-oct-6-enyl acetate: 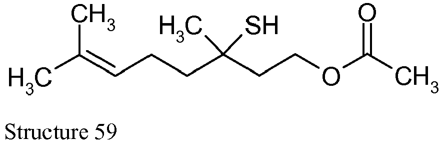
3-Mercapto-3,7-dimethyl-octa-4,6-dienyl acetate: 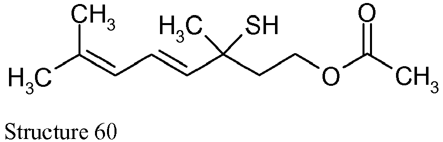
S-[1-(2-Hydroxy-ethyl)-1,5-dimethyl-hex-4-enyl]-thioacetate: 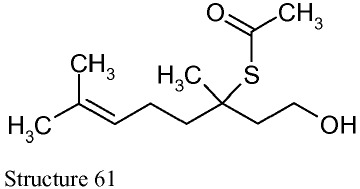
S-[1-(2-Hydroxy-ethyl)-1,5-dimethyl-hexa-2,4-dienyl]-thioacetate: 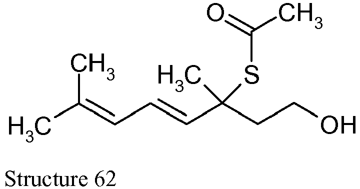
S-[1,5-Dimethyl-1-(2-oxo-ethyl)-hex-4-enyl]-thioacetate: 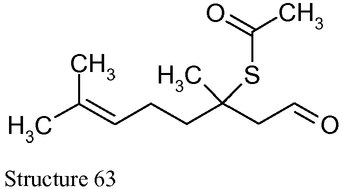
S-[1,5-Dimethyl-1-(2-oxo-ethyl)-hexa-2,4-dienyl]-thioacetate: 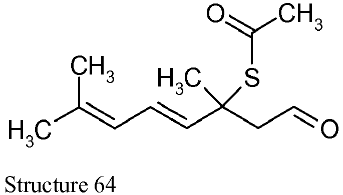
3-Acetylsulfanyl-3,7-dimethyl-oct-6-enyl acetate: 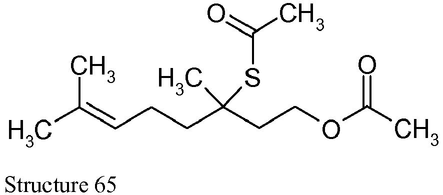
3-Acetylsulfanyl-3,7-dimethyl-octa-4,6-dienyl acetate: 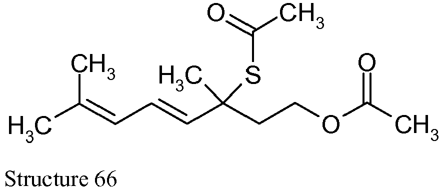
3,7-Dimethyl-oct-6-ene-1,3-dithiol: 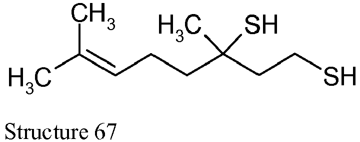
3,7-Dimethyl-1-methylsulfanyl-oct-6-ene-3-thiol: 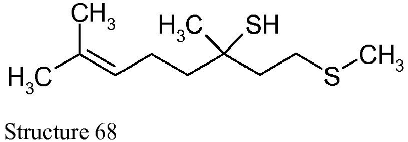
S-(3-Mercapto-3,7-dimethyl-oct-6-enyl)-thioacetate:

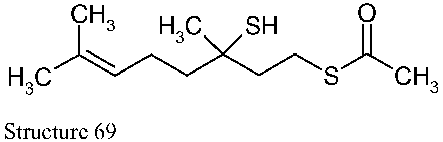

The compounds of the present invention can be prepared, for example, according to the following reaction schemes, the details of which are specified in the Examples. Materials were purchased from Sigma-Aldrich Chemical Company unless noted otherwise.

The thiophen-2-yl compounds of the present invention can be prepared, for example, according to the following procedures: 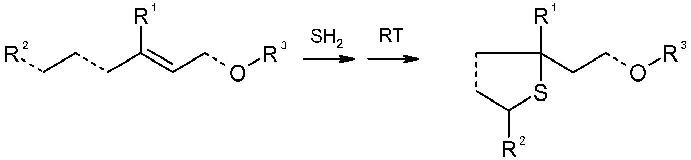
wherein R¹, R² and R³ are defined as above; and
RT represents room temperature.

The thiophen-2-yl compounds of the present invention can be prepared, for example, according to the following procedures: 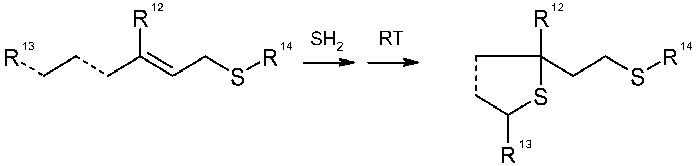
wherein R¹², R¹³ and R¹⁴ are defined as above; and
RT represents room temperature.

The oct-6-enyl compounds of the present invention can be prepared, for example, according to the following procedures: 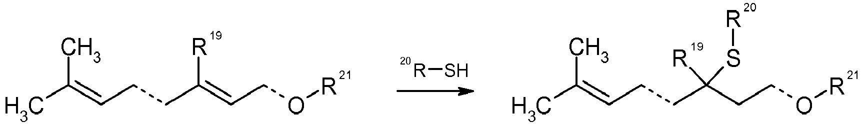 wherein R¹⁹, R²⁰ and R²¹ are defined as above.

The above preparation is detailed in the Examples. Materials were purchased from Aldrich Chemical Company unless noted otherwise.

Those with skill in the art will recognize that some of the compounds of the present invention contain chiral centers, thereby providing a number of isomers of the claimed compounds. It is intended herein that the compounds described herein include isomeric mixtures of such compounds, as well as individual isomers that may be separated using techniques known to those having skill in the art. Suitable techniques include chromatography such as high performance liquid chromatography, referred to as HPLC, particularly silica gel chromatograph, and gas chromatography trapping known as GC trapping. Yet, commercial versions of such products are mostly offered as mixtures.

The compounds of the present invention are found to have unexpected strong and long-lasting organoleptic properties such as taste and odor, which are shown to be advantageous for their use in augmenting or imparting taste enhancement or somatosensory effect to foodstuffs, chewing gums, dental and oral hygiene products and medicinal products by providing flavor enhancement and a preferred overall flavor profile. The present invention further relates to a process of augmenting or imparting taste or somatosensory effect to foodstuffs, chewing gums, dental and oral hygiene products and medicinal products by adding the compounds of the present invention.

When the compounds of the present invention are used in a flavoring composition, they can be combined with conventional flavoring materials or adjuvants, which are well known in the art and have been extensively described in the past. Conventional flavoring materials include saturated fatty acids, unsaturated fatty acids, amino acids; alcohols including primary and secondary alcohols; esters; carbonyl compounds including ketones; aldehydes; lactones; cyclic organic materials including benzene derivatives, acyclic compounds, heterocyclies such as furans, pyridines, pyrazines and the like; sulfur-containing compounds including thiols, sulfides, disulfides and the like; proteins; lipids; carbohydrates; so-called flavor potentiators such as monosodium glutamate; magnesium glutamate, calcium glutamate, guanylates and inosinates; natural flavoring materials such as hydrolyzates, cocoa, vanilla and caramel; essential oils and extracts such as anise oil, clove oil and the like; and artificial flavoring materials such as vanillin, ethyl vanillin and the like. Requirements for adjuvants include: (1) that they be non-reactive with the compounds of the present invention; (2) that they be organoleptically compatible with the compounds of the present invention, whereby the flavor of the ultimate consumable product to which the compounds are added is not detrimentally affected by the use of the adjuvants; and (3) that they be ingestible acceptable and thus nontoxic or otherwise non-deleterious. In addition, other flavor materials, vehicles, stabilizers, thickeners, surface active agents, conditioners and flavor intensifiers can also be included.

The unexpected strong and long-lasting organoleptic properties of the compounds of the present invention further shows their advantageous use in current perfumery products, including the preparation of perfumes and colognes, the perfuming of personal care products such as soaps, shower gels, and hair care products, fabric care products, air fresheners, and cosmetic preparations. The present invention can also be used to perfume cleaning agents, such as, but not limited to detergents, dishwashing materials, scrubbing compositions, window cleaners and the like.

In these preparations, the compounds of the present invention can be used alone or in combination with other perfuming compositions, solvents, adjuvants and the like. The nature and variety of the other ingredients that can also be employed are known to those with skill in the art. Many types of fragrances can be employed in the present invention, the only limitation being the compatibility with the other components being employed. Suitable fragrances include but are not limited to fruits such as almond, apple, cherry, grape, pear, pineapple, orange, strawberry, raspberry; musk, flower scents such as lavender-like, rose-like, iris-like, carnation-like. Other pleasant scents include herbal and woodland scents derived from pine, spruce and other forest smells. Fragrances may also be derived from various oils, such as essential oils, or from plant materials such as peppermint, spearmint and the like.

A list of suitable fragrances is provided in US Pat. No. 4,534,891, the contents of which are incorporated by reference as if set forth in its entirety. Another source of suitable fragrances is found in Perfumes, Cosmetics and Soaps, Second Edition, edited by W. A. Poucher, 1959. Among the fragrances provided in this treatise are acacia, cassie, chypre, cyclamen, fern, gardenia, hawthorn, heliotrope, honeysuckle, hyacinth, jasmine, lilac, lily, magnolia, mimosa, narcissus, freshly-cut hay, orange blossom, orchid, reseda, sweet pea, trefle, tuberose, vanilla, violet, wallflower, and the like.

The compounds of the present invention can be used in combination with a complementary fragrance compound. The term "complementary fragrance compound" as used herein is defined as a fragrance compound selected from the group consisting of 2-[(4-methylphenyl)methylene]-heptanal (Acalea), iso-amyl oxyacetic acid allylester (Allyl Amyl Glycolate), (3,3-dimethylcyclohexyl)ethyl ethyl propane-1,3-dioate (Applelide), (E/Z)-1-ethoxy-1-decene (Arctical), 2-ethyl-4-(2,2,3-trimethyl-3-cyclo-penten-1-yl)-2-buten-1-ol (Bacdanol), 2-methyl-3-[(1,7,7-trimethylbicyclo[2.2.1]hept-2-yl)oxy] exo-1-propanol (Bornafix), 1,2,3,5,6,7-hexahydro-1,1,2,3,3-pentamethyl-4H-inden-4-one (Cashmeran), trimethylcyclopentenylmethyloxabicyclooctane (Cassiffix), 1,1-dimethoxy-3,7-dimethyl-2,6-octadiene (Citral DMA), 3,7-dimethyl-6-octen-1-ol (Citronellol), 3A,4,5,6,7,7A-hexahydro-4,7-methano-1*H*-inden-5/6-yl acetate (Cyclacet), 3A,4,5,6,7,7A-hexahydro-4,7-methano-1*H*-inden-5/6-yl propinoate (Cyclaprop), 3A,4,5,6,7,7A-hexahydro-4,7-methano-1G-inden-5/6-yl butyrate (Cyclobutanate), 1-(2,6,6-trimethyl-3-cyclohexen-1-yl)-2-buten-1-one (Delta Damascone), 3-(4-ethylphenyl)-2,2-dimethyl propanenitrile (Fleuranil), 3-(O/P-ethylphenyl) 2,2-dimethyl propionaldehyde (Floralozone), tetrahydro-4-methyl-2-(2-methylpropyl)-2H-pyran-4-ol (Floriffol), 1,3,4,6,7,8-hexahydro-4,6,6,7,8,8-hexamethylcyclopenta-gamma-2-benzopyran (Galaxolide), 1-(5,5-dimethyl-1-cyclohexen-1-yl)pent-4-en-1-one (Galbascone), E/Z-3,7-dimethyl-2,6-octadien-1-yl acetate (Geranyl Acetate), α-methyl-1,3-benzodioxole-5-propanal (Helional), 1-(2,6,6-trimethyl-2-cyclohexen-1-yl)-1,6-heptadien-3-one (Hexalon), (Z)-3-hexenyl-2-hydroxybenzoate (Hexenyl Salicylate, CIS-3), 4-(2,6,6-trimethyl-2-cyclohexen-1-yl)-3-buten-2-one (Ionone a), 1-(1,2,3,4,5,6,7,8-octahydro-2,3,8,8-tetramethyl-2-naphthalenyl)-ethan-1-one (Iso E Super), methyl 3-oxo-2-pentylcyclopentaneacetate (Kharismal), 2,2,4-trimethyl-4-phenyl-butanenitrile (Khusinil), 3,4,5,6,6-pentamethylhept-3-en-2-one (Koavone), 3/4-(4-hydroxy-4-methyl-pentyl) cyclohexene-1-carboxaldehyde (Lyral), 3-methyl-4-(2,6,6-trimethyl-2-cyclohexen-1-yl)-3-buten-2-one (Methyl Ionone γ), 1-(2,6,6-trimethyl-2-cyclohexen-1-yl) pent-1-en-3-one (Methyl Ionone α Extra, Methyl Ionone N), 3-methyl-4-phenylbutan-2-ol (Muguesia), cyclopentadec-4-en-1-one (Musk Z4), 3,3,4,5,5-pentamethyl-11,13-dioxatricyclo[7.4.0.0<2,6>]tridec-2(6)-ene (Nebulone), 3,7-dimethyl-2,6-octadien-1-yl acetate (Neryl Acetate), 3,7-dimethyl-1,3,6-octatriene (Ocimene), ortho-tolylethanol (Peomosa), 3-methyl-5-phenylpentanol (Phenoxanol), 1-methyl-4-(4-methyl-3-pentenyl) cyclohex-3-ene-1-carboxaldehyde (Precyclemone B), 4-methyl-8-methylene-2-adamantanol (Prismantol), 2-ethyl-4-(2,2,3-trimethyl-3-cyclopenten-1-yl)-2-buten-1-ol (Sanjinol), 2-methyl-4-(2,2,3-trimethyl-3-cyclopenten-1-yl)-2-buten-1-ol (Santaliff), Terpineol, 2,4-dimethyl-3-cyclohexene-1-carboxaldehyde (Triplal), decahydro-2,6,6,7,8,8-hexamethyl-2H-indeno[4,5-B]furan (Trisamber), 2-tert-butylcyclohexyl acetate (Verdox), 4-tert-butylcyclohexyL acetate (Vertenex), acetyl cedrene (Vertofix), 3,6/4,6-dimethylcyclohex-3-ene-1-carboxaldehyde (Vertoliff), and (3Z)-1-[(2-methyl-2-propenyl)oxy]-3-hexene (Vivaldie).

The terms "flavor composition" and "flavor formulation" mean the same and refer to a consumer composition that produces a pleasant or desired flavor. The flavor composition contains a compound or a mixture of compounds. The flavor composition of the present invention is a consumer composition comprising a compound of the present invention.

The term "foodstuff" as used herein includes both solid and liquid ingestible materials for man or animals, which materials usually do, but need not, have nutritional value. Thus, foodstuffs include meats, gravies, soups, convenience foods, malt, alcoholic and other beverages, milk and dairy products, seafood, including fish, crustaceans, mollusks and the like, candies, vegetables, cereals, soft drinks, snacks, dog and cat foods, other veterinary products and the like.

The terms "fragrance composition", "fragrance formulation" and "perfume composition" mean the same and refer to a consumer composition that is a mixture of compounds including, for example, alcohols, aldehydes, ketones, esters, ethers, lactones, nitriles, natural oils, synthetic oils, and mercaptans, which are admixed so that the combined odors of the individual components produce a pleasant or desired fragrance. The fragrance composition of the present invention is a consumer composition comprising a compound of the present invention. The fragrance composition of the present invention comprises a compound of the present invention and further a complementary fragrance compound as defined above.

The term "fragrance product" means a consumer product containing a fragrance ingredient that adds fragrance or masks malodor. Fragrance products may include, for example, perfumes, colognes, bar soaps, liquid soaps, shower gels, foam baths, cosmetics, skin care products such as creams, lotions and shaving products, hair care products for shampooing, rinsing, conditioning, bleaching, coloring, dyeing and styling, deodorants and antiperspirants, feminine care products such as tampons and feminine napkins, baby care products such as diapers, bibs and wipes, family care products such as bath tissues, facial tissues, paper handkerchiefs or paper towels, fabric products such as fabric softeners and fresheners, air care products such as air fresheners and fragrance delivery systems, cosmetic preparations, cleaning agents and disinfectants such as detergents, dishwashing materials, scrubbing compositions, glass and metal cleaners such as window cleaners, countertop cleaners, floor and carpet cleaners, toilet cleaners and bleach additives, washing agents such as all-purpose, heavy duty, and hand washing or fine fabric washing agents including laundry detergents and rinse additives, dental and oral hygiene products such as toothpastes, tooth gels, dental flosses, denture cleansers, denture adhesives, dentifrices, tooth whitening and mouthwashes, health care and nutritional products and food products such as snack and beverage products. The fragrance product of the present invention is a consumer product that contains a compound of the present invention. The fragrance product of the present invention contains a compound of the present invention and further a complementary fragrance compound as defined above.

The term "improving" is understood to mean raising a flavor or fragrance composition to a more desirable character. The term "enhancing" is understood to mean making the flavor or fragrance composition greater in effectiveness or providing the flavor or fragrance composition with an improved character. The term "modifying" is understood to mean providing the flavor or fragrance composition with a change in character.

The term "olfactory acceptable amount" is understood to mean the amount of a compound in a flavor or fragrance composition, wherein the compound will contribute its individual olfactory characteristics. However, the olfactory effect of the flavor or fragrance composition will be the sum of effect of each of the flavor or fragrance ingredients. Thus, the compound of the present invention can be used to improve or enhance the aroma characteristics of the flavor or fragrance composition, or by modifying the olfactory reaction contributed by other ingredients in the composition. The olfactory acceptable amount may vary depending on many factors including other ingredients, their relative amounts and the olfactory effect that is desired.

Generally, the olfactory acceptable amount of the compounds employed in a flavor composition is greater than about 0.1 parts per billion by weight, preferably from about 1 part per billion to about 500 parts per million by weight, more preferably from about 10 parts per billion to about 100 parts per million by weight, even more preferably from about 100 parts per billion to about 50 parts per million by weight. The olfactory acceptable amount of the compounds employed in a fragrance composition varies from about 0.005 to about 70 weight percent, preferably from 0.005 to about 50 weight percent, more preferably from about 0.5 to about 25 weight percent, and even more preferably from about 1 to about 10 weight percent. Those with skill in the art will be able to employ the desired amount to provide desired flavor or fragrance effect and intensity. In addition to the compounds of the present invention, other materials can also be used in conjunction with the flavor or fragrance composition to encapsulate and/or deliver the flavor or fragrance. Some well-known materials are, for example, but not limited to, polymers, oligomers, other non-polymers such as surfactants, emulsifiers, lipids including fats, waxes and phospholipids, organic oils, mineral oils, petrolatum, natural oils, perfume fixatives, fibers, starches, sugars and solid surface materials such as zeolite and silica.

In addition, the compounds of the present invention may also provide superior ingredient performance and possess unexpected advantages in malodor counteracting applications such as body perspiration, environmental odor such as mold and mildew, bathroom, and etc. The compounds of the present invention substantially eliminate the perception of malodors and/or prevent the formation of such malodors, thus, can be utilized with a vast number of functional products.

Examples of the functional products are provided herein to illustrate the various aspects of the present invention. However, they do not intend to limit the scope of the present invention. The functional products may include, for example, a conventional room freshener (or deodorant) composition such as room freshener sprays, an aerosol or other spray, fragrance diffusers, a wick or other liquid system, or a solid, for instance candles or a wax base as in pomanders and plastics, powders as in sachets or dry sprays or gels, as in solid gel sticks, clothes deodorants as applied by washing machine applications such as in detergents, powders, liquids, whiteners or fabric softeners, fabric refreshers, linen sprays, closet blocks, closet aerosol sprays, or clothes storage areas or in dry cleaning to overcome residual solvent notes on clothes, bathroom accessories such as paper towels, bathroom tissues, sanitary napkins, towellets, disposable wash cloths, disposable diapers, and diaper pail deodorants, cleansers such as disinfectants and toilet bowl cleaners, cosmetic products such as antiperspirant and deodorants, general body deodorants in the form of powders, aerosols, liquids or solid, or hair care products such as hair sprays, conditioners, rinses, hair colors and dyes, permanent waves, depilatories, hair straighteners, hair groom applications such as pomade, creams and lotions, medicated hair care products containing such ingredients as selenium sulphide, coal tar or salicylates, or shampoos, or foot care products such as foot powders, liquids or colognes, after shaves and body lotions, or soaps and synthetic detergents such as bars, liquids, foams or powders, odor control such as during manufacturing processes, such as in the textile finishing industry and the printing industry (inks and paper), effluent control such as in processes involved in pulping, stock yard and meat processings, sewage treatment, garbage bags, or garbage disposal, or in product odor control as in textile finished goods, rubber finished goods or car fresheners, agricultural and pet care products such as dog and hen house effluents and domestic animal and pet care products such as deodorants, shampoo or cleaning agents, or animal litter material and in large scale closed air systems such as auditoria, and subways and transport systems.

Thus, it will be seen that the composition of the invention is usually one in which the malodor counteractant is present together with a carrier by means of which or from which the malodor counteractant can be introduced into air space wherein the malodor is present, or a substrate on which the malodor has deposited. For example, the carrier can be an aerosol propellant such as a chlorofluoro-methane, or a solid such as a wax, plastics material, rubber, inert powder or gel. In a wick-type air freshener, the carrier is a substantially odorless liquid of low volatility. In several applications, a composition of the invention contains a surface active agent or a disinfectant, while in others, the malodor counteractant is present on a fibrous substrate. In many compositions of the invention there is also present a fragrance component which imparts a fragrance to the composition. The fragrances stated above can all be employed.

Malodor counteracting effective amount is understood to mean the amount of the inventive malodor counteractant employed in an air space or a substrate such as a functional product that is organoleptically effective to abate a given malodor while reducing the combined intensity of the odor level, wherein the given malodor is present in air space or has deposited on a substrate. The exact amount of malodor counteractant agent employed may vary depending upon the type of malodor counteractant, the type of the carrier employed, and the level of malodor counteractancy desired. In general, the amount of malodor counteractant agent present is the ordinary dosage required to obtain the desired result. Such dosage is known to the skilled practitioner in the art. In a preferred embodiment, when used in conjunction with malodorous solid or liquid functional products, e.g., soap and detergent, the compounds of the present invention may be present in an amount ranging from about 0.005 to about 50 weight percent, preferably from about 0.01 to about 20 weight percent, more preferably from about 0.05 to about 10 weight percent and even more preferably from about 0.1 to about 5 weight percent. When used in an air space that is in conjunction with malodorous gaseous functional products, the compounds of the present invention may be present in an amount ranging from about 0.2 mg to about 2 g per cubic meter of air, more preferably from about 0.4 mg to about 0.8 g per cubic meter of air, more preferably from about 2 mg to about 0.4 g per cubic meter of air and even more preferably from about 4 mg to about 0.2 g per cubic meter of air.

The following are provided as specific embodiments of the present invention. Other modifications of this invention will be readily apparent to those skilled in the art. Such modifications are understood to be within the scope of this invention. As used herein all percentages are weight percent unless otherwise noted, ppb is understood to stand for parts per billion, ppm is understood to stand for parts per million, L is understood to be liter, mL is understood to be milliliter, Kg is understood to be kilogram, g is understood to be gram, mol is understood to be mole and M is understood to be molar. IFF as used in the examples is understood to mean International Flavors & Fragrances Inc., New York, NY, USA.

### EXAMPLE I

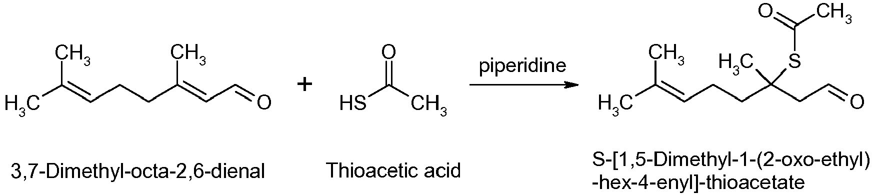

**Preparation of S-[1,5-Dimethyl-1-(2-oxo-ethyl)-hex-4-enyl]-thioacetate (Structure 63):** A 1L four-necked flask equipped with a mechanical stirrer, a thermometer, a reflux condenser and an addition funnel was charged with 3,7-dimethyl-octa-2,6-dienal (100 g, 0.66 mol) under an inert atmosphere. Piperidine (0.5 mL) was added followed by drop wise addition of thioacetic acid (60 g, 0.79 mol) at a temperature ranging between 0-10 °C. The reaction mixture was then stirred at room temperature for 18 hours. The reaction mixture was diluted with hexane (100 mL) and subsequently washed with hydrochloric acid (HC1) (1 M, 200 mL), twice with saturated sodium bicarbonate solution (NaHCO₃) (200 mL), twice with water (200 mL) and brine (200 mL). The solvent was then evaporated and raw oil was distilled to separate unreacted materials. The crude product was obtained at 108 °C and 1.9 Torr (30 g).

¹H NMR (CDCl₃, 500-MHz): 9.80 ppm (t, J=2.5 Hz, 1H), 4.98-5.13 ppm (m, 1H), 3.06 ppm (dd, J=15.9 Hz, 2.5 Hz, 1H), 2.90 ppm (dd, J=15.9 Hz, 2.5 Hz, 1H), 2.27 ppm (s, 3H), 1.97-2.11 ppm (m, 2H), 1.84-1.95 ppm (m, 1H), 1.73 ppm (m, 1H), 1.67 ppm (s, 3H), 1.59 ppm (s, 3H), 1.53 ppm (s, 3H)

S-[1,5-Dimethyl-1-(2-oxo-ethyl)-hex-4-enyl]-thioacetate was described as having lemon, grapefruit, tropical and passion fruit organoleptic notes.

### EXAMPLE II

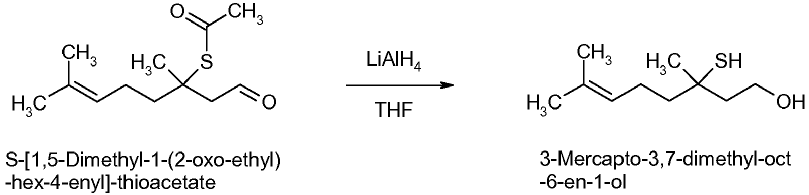

**Preparation of 3-Mercapto-3,7-dimethyl-oct-6-en-1-ol (Structure 55):** Lithium aluminum hydride (LiAlH₄) (3.32 g, 88 mmol) was dissolved in tetrahydrofuran (THF) (200 mL) at room temperature and then cooled to 0 °C with a cooling bath. S-[1,5-Dimethyl-1-(2-oxo-ethyl)-hex-4-enyl]-thioacetate (obtained as above in EXAMPLE I, 20 g, 88 mmol) was added drop wise to the reaction mixture under nitrogen while the temperature was kept below 10 °C. After the addition was completed, the cooling bath was removed. The reaction mixture was stirred at room temperature for additional 30 minutes and then poured into ammonium chloride solution (200 mL), which was then transferred to a separatory funnel and extracted with methyl *t*-butyl ether (400 mL). The organic layer was washed with brine twice. The solvent was removed under vacuum. The crude was purified by silica column chromatography using ethyl acetate/hexanes (weight ratio of 80:20) eluent to provide 3-mercapto-3,7-dimethyl-oct-6-en-1-ol (8.3 g).

¹H NMR (CDCl₃, 500-MHz): 5.10 ppm (t, J=6.6 Hz, 1H), 3.80-3.89 ppm (m, 2H), 2.01-2.17 (m, 4H), 1.82-1.95 ppm (m, 2H), 1.69 ppm (s, 3H), 1.62 ppm (s, 3H), 1.55-1.70 ppm (m, 2H), 1.38 ppm (s, 3H)

3-Mercapto-3,7-dimethyloct-6-en-1-ol was described as having lemon, green and citrusy organoleptic notes.

### EXAMPLE III

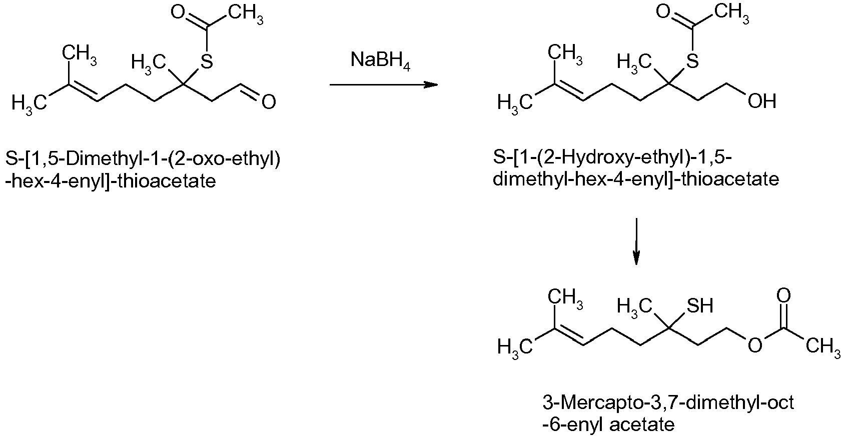

**Preparation of 3-Mercapto-3,7-dimethyl-oct-6-enyl Acetate (Structure 59):** Sodium borohydride (NaBH₄) (2.5 g, 66 mmol) was dissolved in ethanol (40 mL) and water (10 mL). Temperature of the reaction mixture was lowered to 0 °C with a cooling bath. S-[1,5-Dimethyl-1-(2-oxo-ethyl)-hex-4-enyl]-thioacetate (obtained as above in EXAMPLE I, 10 g, 43.8 mmol) was added drop wise to the reaction mixture under nitrogen while the temperature was kept below 10 °C. After the addition was completed, the cooling bath was removed. The reaction mixture was stirred at room temperature for additional 30 minutes. Intermediate S-[1-(2-hydroxy-ethyl)-1,5-dimethyl-hex-4-enyl]-thioacetate (Structure 61) was produced but converted instantly to product 3-mercapto-3,7-dimethyl-oct-6-enyl acetate. The reaction mixture was poured into ammonium chloride solution (100 mL) and transferred to a separatory funnel and then extracted with methyl *t*-butyl ether (200 mL). The organic layer was washed with brine twice. The solvent was removed under vacuum. The crude was purified by silica column chromatography using ethyl acetate/hexanes (weight ratio of 10:90) eluent to provide final product 3-mercapto-3,7-dimethyl-oct-6-enyl acetate (8.2 g).

¹H NMR (CDCl₃, 400-MHz): 5.00-5.14 ppm (m, 1H), 4.21-4.30 ppm (m, 2H), 2.07-2.15 ppm (m, 2H), 2.04 ppm (s, 3H), 1.92-1.96 ppm (m, 2H), 1.68 ppm (s, 3H), 1.62 ppm (s, 3H) ,1.56-1.70 ppm (m, 3H), 1.38 ppm (s, 3H)

3-Mercapto-3,7-dimethyl-oct-6-enyl acetate was described has having juicy grapefruit and mandarin organoleptic notes.

### EXAMPLE IV

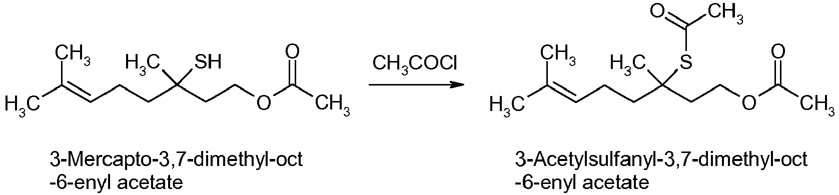

**Preparation of 3-Acetylsulfanyl-3,7-dimethyl-oct-6-enyl Acetate (Structure 65):** 3-Mercapto-3,7-dimethyl-oct-6-enyl acetate (obtained as above in EXAMPLE III, 4.61 g, 20 mmol) and triethylamine ((CH₃)₃N) (2.23 g, 22 mmol) were dissolved in dichloromethane (40 mL). The temperature of the reaction mixture was lowered to 0 °C with a cooling bath. Acetyl chloride (CH₃COCl) (1.73 g, 22 mmol) was added drop wise to the reaction mixture. After the addition was completed, the cooling bath was removed. The reaction mixture was stirred at room temperature for additional 2 hours, transferred to a separatory funnel and then washed with brine (10 mL), hydrochloric acid (1 M, 10 mL) and brine (10 mL). The solvent was removed under vacuum. The crude was purified on a silica column using ethyl acetate/hexanes to provide product 3-acetylsulfanyl-3,7-dimethyl-oct-6-enyl acetate (4.9 g).

¹H NMR (CDCl₃, 500-MHz): 5.06 ppm (m, 1H), 4.17 ppm (t, J=7.3 Hz, 2H), 2.24 ppm (s, 3H), 2.22-2.28 ppm (m, 1H), 2.09-2.18 ppm (m, 1H), 2.03 ppm (s, 3H), 1.92-2.07 ppm (m, 2H), 1.77-1.87 ppm (m, 1H), 1.67 ppm (s, 3H), 1.64-1.72 ppm (m, 1H), 1.60 ppm (s, 3H), 1.44 ppm (s, 3H)

3-Acetylsulfanyl-3,7-dimethyl-oct-6-enyl acetate was described as having sulfury, tropical and ripe orange organoleptic notes.

### EXAMPLE V

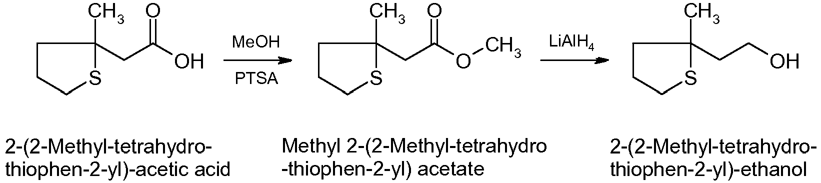

**Preparation of 2-(2-Methyl-tetrahydro-thiophen-2-yl)-ethanol (Structure 1):** 2-(2-Methyl-tetrahydro-thiophen-2-yl)-acetic acid (5.1 g, 32 mmol), prepared according to the procedure described previously (*See,* Bunce, et al. J. Org. Chem. (1992) 57:1727-1733), was dissolved in Methanol (MeOH) (100 mL). *p*-Toluenesulfonic acid monohydrate (PTSA) (0.6 g, 3 mmol) was added and the reaction mixture was refluxed for 3 hours. The solvent was removed under vacuum and ethyl acetate (EtOAc) (100 mL) was then added. The organic layer was washed with sodium bicarbonate solution (5%, 100 mL) followed by brine (100 mL), and dried over magnesium sulfate (MgSO₄). Further filtration and concentration provided crude methyl 2-(2-methyl-tetrahydro-thiophen-2-yl) acetate (5.0 g). Lithium aluminium hydride (LiAlH₄) (1.1 g, 29 mmol) was dissolved in THF (100 mL) and charged into a reaction flask. Under N₂, a solution of crude methyl 2-(2-methyl-tetrahydro-thiophen-2-yl) acetate (5.0 g, 29 mmol) in THF (40 mL) was added dropwise into the reaction flask. The reaction mixture was then allowed to be stirred for additional 2 hours at room temperature. The resulting mixture was added to a solution of ammonium chloride (NH₄Cl) (10%, 200 mL) and extracted with ether (200 mL). The organic layer was washed with brine (100 mL) and dried over MgS04. Filtration, concentration and further purification with silica column chromatography using ethyl acetate/hexanes provided product 2-(2-methyl-tetrahydro-thiophen-2-yl)-ethanol (3.8 g).

¹H NMR (CDCl₃, 500-MHz): 3.74-3.89 (m, 2H), 2.87-3.03 (m, 2H), 2.39 (br s, 1H), 2.00-2.16 (m, 2H), 1.71-1.98 (m, 4H), 1.41 (s, 2H), 1.36-1.44 (m, 1H)

### EXAMPLE VI

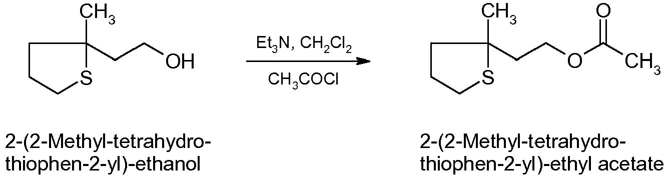

**Preparation of 2-(2-Methyl-tetrahydro-thiophen-2-yl)-ethyl acetate (Structure 7):** 2-(2-Methyl-tetrahydro-thiophen-2-yl)-ethanol (obtained as above in EXAMPLE V, 3.8 g, 26 mmol) and triethylamine (Et₃N)(3.2 g, 32 mmol) were dissolved in dichloromethane (CH₂Cl₂) (100 mL), charged into a reaction flask and cooled to 0 °C. With stirring, a solution of acetyl chloride (CH₃COCl) (2.5 g, 32 mmol) in CH₂Cl₂ (20 mL) was added dropwise to the reaction flask. The reaction mixture was then stirred for additional 2 hours at room temperature. The resulting mixture was transferred to a separatory funnel and washed with a solution of NH₄Cl (10%, 100 mL), a solution of NaHCO₃ (5%, 100 mL), brine (100 mL) and then dried over MgSO₄. Filtration, concentration and further purification with silica column chromatography using ethyl acetate/hexanes provided product 2-(2-methyl-tetrahydro-thiophen-2-yl)-ethyl acetate.

¹H NMR (CDCl₃, 400-MHz): 4.17-4.29 (m, 2H), 2.88-3.01 (m, 2H), 1.98-2.12 (m, 4H), 2.05 (s, 3H), 1.73-1.89 (m, 2H), 1.42 (s, 3H).

2-(2-Methyl-tetrahydro-thiophen-2-yl)-ethyl acetate was described as having slightly fruity, slight tropical, citrus, floral, green and sulfury organoleptic notes.

### EXAMPLE VII

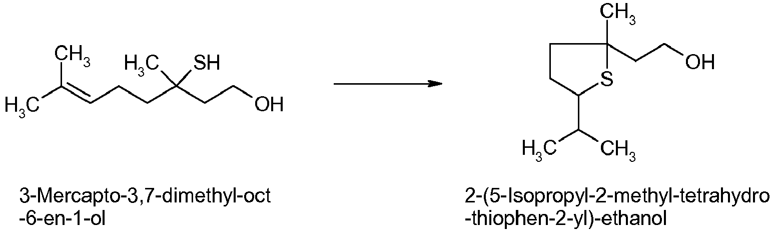

**Preparation of 2-(5-Isopropyl-2-methyl-tetrahydro-thiophen-2-yl)-ethanol (Structure 10):** 3-Mercapto-3,7-dimethyl-oct-6-en-1-ol (obtained as above in EXAMPLE II, 8.3 g) underwent internal cyclisation at room temperature for 10 days to provide 2-(5-isopropyl-2-methyl-tetrahydro-thiophen-2-yl)-ethanol (8.0 g).

¹H NMR (CDCl₃, 500-MHz): 3.72-3.86 ppm (m, 2H), 3.24-3.32 ppm (m, 1H), 2.59 ppm (br, 1H), 1.56-2.18 ppm (m, 7H), 1.41 ppm (s, ∼ 50% of 3H), 1.38 ppm (s, ∼ 50% of 3H), 0.92-0.98 ppm (m, 6H)

2-(5-Isopropyl-2-methyl-tetrahydro-thiophen-2-yl)-ethanol was described as having lime, tropical and leafy green organoleptic notes.

### EXAMPLE VIII

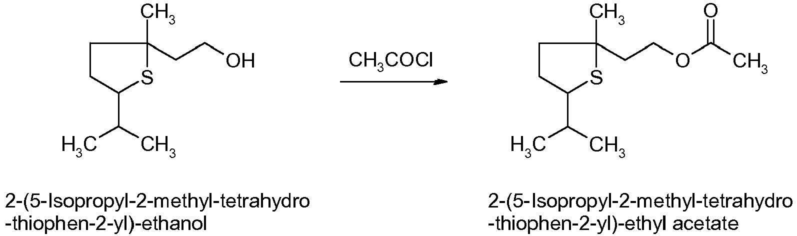

**Preparation of 2-(5-Isopropyl-2-methyl-tetrahydro-thiophen-2-yl)-ethyl Acetate (Structure 16):** A 50 mL flask equipped with a magnetic stirrer and a thermometer was charged with 2-(5-isopropyl-2-methyl-tetrahydro-thiophen-2-yl)-ethanol (obtained as above in EXAMPLE VII, 600 mg, 3.2 mmol) and dichloromethane (CH₂Cl₂) (5 mL). The reaction mixture was cooled to 0 °C. Triethylamine (500 mg, 4.8 mmol) was added followed by acetyl chloride (280 mg, 3.5 mmol). After an hour, hydrochloric acid (1 M) (5 mL) was added and the reaction was extracted twice with dichloromethane. The reaction mixture was then washed with water (10 mL) and brine (10 mL) and dried over anhydrous magnesium sulfate (MgSO₄). The solvent was evaporated and the crude product was purified by silica column chromatography using ethyl acetate/hexanes (weight ratio of 95:5) eluent. The product 2-(5-isopropyl-2-methyl-tetrahydro-thiophen-2-yl)-ethyl acetate was obtained as an oil (500 mg).

¹H NMR (CDCl₃, 500-MHz): 4.12-4.33 ppm (m, 2H), 3.17-3.36 ppm (m, 1H), 2.07-2.20 ppm (m, 1H), 2.04 ppm (s, 3H), 1.64-2.03 ppm (m, 6H), 1.42 ppm (s, ∼ 50% of 3H), 1.40 ppm (s, ∼ 50% of 3H), 0.91-0.98 ppm (m, 6H)

2-(5-Isopropyl-2-methyl-tetrahydro-thiophen-2-yl)-ethyl acetate was described as having tropical, strawberry and green pepper organoleptic notes.

### EXAMPLE IX

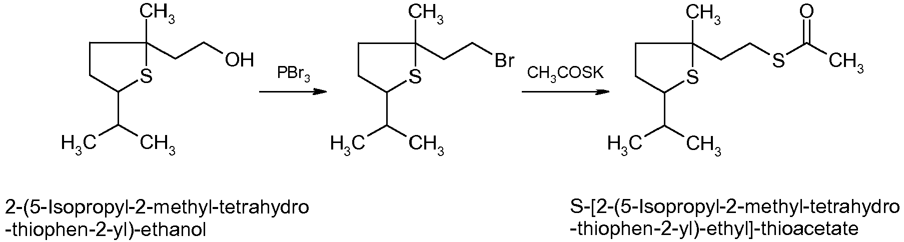

**Preparation of S-[2-(5-Isopropyl-2-methyl-tetrahydro-thiophen-2-yl)-ethyl] -thioacetate: (Structure 52):** Phosphorus tribromide (PBr₃) (19 g, 70 mmol) was added to 2-(5-isopropyl-2-methyl-tetrahydro-thiophen-2-yl)-ethanol (40 g, 0.212 mol) dropwise at 10 °C and the reaction mixture was aged for 1 hour. Diethyl ether ((C₂H₅)₂O) (100 mL) and brine (50 mL) were then added. The organic layer was separated and concentrated via vacuum, and was subsequently dissolved in dimethylformamide (DMF) (250 mL) and combined with potassium thioacetate (CH₃COSK) (32.7 g, 0.287 mol). The reaction mixture was heated to 80 °C for 1 hour and then cooled to room temperature. Water (400 mL) was added and the aqueous mixture was extracted with diethyl ether. The organic layer was concentrated via vacuum and fractionally distilled to provide S-[2-(5-isopropyl-2-methyl-tetrahydro-thiophen-2-yl)-ethyl]-thioacetate (19 g).

¹H NMR (CDCl₃, 500-MHz): 3.17-3.37 (m, 1H), 2.83-3.08 (m, 2H), 2.32 (s, 45% of 3H), 2.32 (s, 55% of 3H), 2.08-2.20 (m, 1H), 1.60-2.02 (m, 6H), 1.44 (s, 45% of 3H), 1.41 (s, 55% of 3H), 0.90-0.99 (m, 6H)

2-(5-Isopropyl-2-methyl-tetrahydro-thiophen-2-yl)-ethyl acetate was described as having powerful, tropical, fruity, zesty, fresh, vegetative, burnt sugar, nutty, sesame and bacon organoleptic notes.

### EXAMPLE X

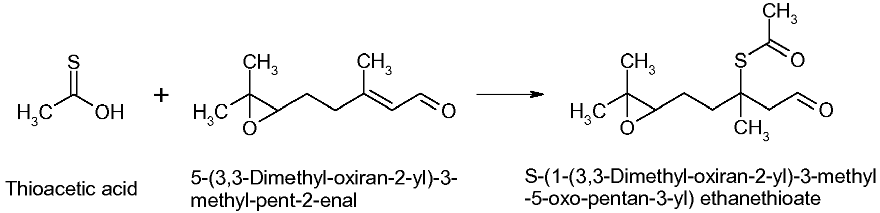

**Preparation of S-(1-(3,3-Dimethyl-oxiran-2-yl)-3-methyl-5-oxo-pentan-3-yl) ethanethioate:** Thioacetic acid (4.0 g, 53 mmol) and 5-(3,3-dimethyl-oxiran-2-yl)-3-methyl-pent-2-enal (8.9 g, 53 mmol) were combined and stirred at 60 °C for 5 hours. The reaction mixture was washed with a solution of NaHCO₃ (5%, 80 mL) and dried over MgSO₄. Purification with silica column chromatography using ethyl acetate/hexanes (weight ratio of 1:3) provided S-(1-(3,3-dimethyl-oxiran-2-yl)-3-methyl-5-oxo-pentan-3-yl) ethanethioate (1.0 g).

¹H NMR (CDCl₃, 400-MHz): 9.79-9.82 (m, 1H), 3.02-3.20 (m, 1H), 2.84-2.95 (m, 1H), 2.64-2.73 (m, 1H), 2.28 (s, 50% of 3H), 2.27 (s, 50% of 3H), 2.02-2.17 (m, 1H), 1.80-1.95 (m, 1H), 1.50-1.72 (m, 2H), 1.53 (s, 50% of 3H), 1.50 (s, 50% of 3H), 1.30 (s, 3H), 1.27 (s, 50% of 3H), 1.26 (s, 50% of 3H)

### EXAMPLE XI

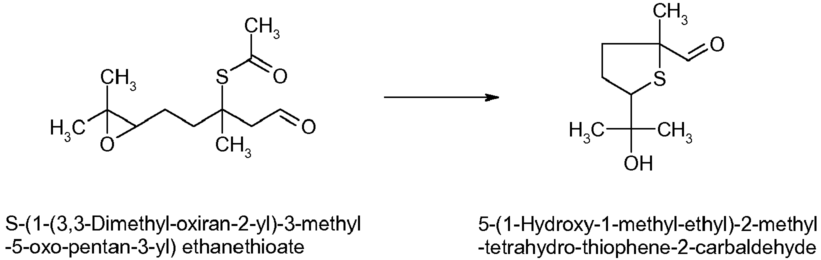

**Preparation of 5-(1-Hydroxy-1-methyl-ethyl)-2-methyl-tetrahydro-thiophene-2-carbaldehyde (Structure 34a):** S-(1-(3,3-Dimethyl-oxiran-2-yl)-3-methyl-5-oxo-pentan-3-yl) ethanethioate (obtained as above in EXAMPLE X, 1.0 g, 4.1 mmol) was dissolved in THF (100 mL) and cooled to 0 °C. Aqueous sodium hydroxide (NaOH) (0.5 M, 8 mL) was added dropwise and the reaction mixture was stirred at 0 °C for about 10 minutes. The reaction was washed with a solution of NH₄Cl (10%, 50 mL) and dried over MgS04. The solvent was removed under vacuum. Purification with silica column chromatography using ethyl acetate/hexanes (weight ratio of 1:3) provided product 5-(1-hydroxy-1-methyl-ethyl)-2-methyl-tetrahydro-thiophene-2-carbaldehyde (0.6 g).

¹H NMR (CDCl₃, 400-MHz): 9.82 (d, J=2.0 Hz, 50% of 1H), 9.81 (d, J=2.0 Hz, 50% of 1H), 3.62-3.84 (m, 1H), 2.60-2.90 (m, 2H), 2.21-2.49 (m, 1H), 1.71-2.18 (m, 4H), 1.56 (s, 50% of 3H), 1.49 (s, 50% of 3H), 1.25 (s, 50% of 3H), 1.23 (s, 50% of 3H), 1.21 (s, 50% of 3H), 1.20 (s, 50% of 3H)

5-(1-Hydroxy-1-methyl-ethyl)-2-methyl-tetrahydro-thiophene-2-carbaldehyde was described as having fresh, green, herbal and earthy but sulfury and weak organoleptic notes.

### EXAMPLE XII

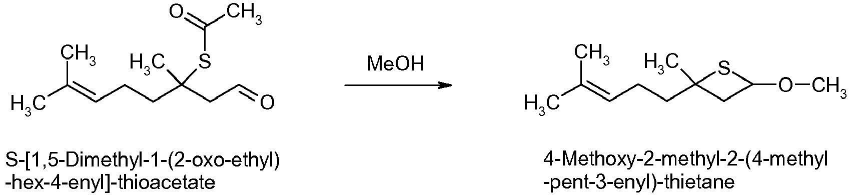

**Preparation of 4-Methoxy-2-methyl-2-(4-methyl-pent-3-enyl)-thietane (Structure 72):** S-[1,5-Dimethyl-1-(2-oxo-ethyl)-hex-4-enyl]-thioacetate (obtained as above in EXAMPLE I, 5 g, 21.9 mmol,\) was dissolved in methanol (CH₃OH) (100 mL) and cooled down to 0 °C with a cooling bath. Potassium hydroxide (KOH) (1.48 g, 26.3 mmol) was dissolved in water (5 mL) and added drop wise to the reaction mixture. After the addition was completed, the reaction mixture was stirred at 0 °C for 10 minutes, transferred to a separatory funnel, and hydrochloric acid (1 M, 50 mL) was added. The reaction mixture was extracted with methyl *t*-butyl ether (100 mL). The organic layer was washed with brine twice and dried with magnesium sulfate. The solvent was removed under vacuum. The crude was purified by silica column chromatography using ethyl acetate/hexanes (weight ratio of 95:5) eluent to provide 4-methoxy-2-methyl-2-(4-methyl-pent-3-enyl)-thietane (2.1 g).

¹H NMR (CDCl₃, 500-MHz): 5.12-5.18 ppm (m, 1H), 5.09-5.13 ppm (m, ∼40% of 1H), 5.00-5.04 ppm (m, ∼60% of 1H), 3.27 ppm (s, ∼60% of 3H), 3.25 ppm (s, ∼40% of 3H), 3.05 ppm (dd, J=12.8 Hz, 7.1 Hz, ∼60% of 1H), ), 2.95 ppm (dd, J=12.7 Hz, 7.3 Hz, ∼40% of 1H), 2.69 ppm (dd, J=12.7 Hz, 4.3 Hz, ∼40% of 1H), 2.60 ppm (dd, J=12.8, 3.6 Hz, ∼60% of 1H), 1.93-2.13 ppm (m, 2H), 1.72-1.93 ppm (m, 2H), 1.69 ppm (s, ∼ 60% of 3H), 1.68 ppm (s, ∼40% of 3H), 1.63 ppm (s, ∼ 60% of 3H), 1.62 ppm (s, ∼40% of 3H), 1.57 ppm (s, ∼ 60% of 3H), 1.54 ppm (s, ∼40% of 3H)

4-Methoxy-2-methyl-2-(4-methyl-pent-3-enyl)-thietane was described as having lemon, lime and sweet brown organoleptic notes.

### EXAMPLE XIII

In addition, the following compounds were similarly prepared according to the procedures described in the above.
2-(5-Isopropyl-2-methyl-2,3-dihydro-thiophen-2-yl)-ethanol (Structure 12) was described as having grapefruit peel, zesty, citrus peel, green, fresh and floral organoleptic notes.
2-[5-(1-Hydroxy-1-methyl-ethyl)-2-methyl-tetrahydro-thiophen-2-yl] -ethyl acetate (Structure 34b) was described as having leafy green but weak and sulfuric organoleptic notes.
2-(5-Isopropenyl-2-methyl-tetrahydro-thiophen-2-yl)-ethyl acetate (Structure 32) was described as having leafy, minty, fresh, juicy but earthy organoleptic notes.
2-(5-Isopropyl-2-methyl-tetrahydro-thiophen-2-yl)-ethanethiol (Structure 43) was described as having leafy, mushroom, sweet, roasted but sulfury and raw organoleptic notes.

## Claims

1. A compound represented by Formula I: 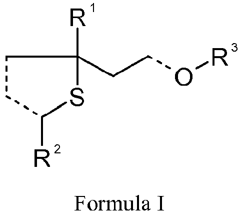 wherein
R¹ is a C₁-C₄ alkyl group;
R² is selected from the group consisting of hydrogen, a C₁-C₄ alkyl group optionally containing a hydroxyl group and a C₁-C₄ alkenyl group optionally containing a hydroxyl group;
R³ is selected from the group consisting of hydrogen, a C₁-C₄ alkyl group and - C(O)R⁴, wherein R⁴ is selected from the group consisting of hydrogen and a C₁-C₄ alkyl group;
one of the dotted lines in the ring represents an optional carbon-carbon double bond; and the dotted line in the side chain represents an optional carbon-oxygen double bond,
with the proviso that, when the optional carbon-oxygen double bond is present, R³ is absent.

2. The compound of claim 1 represented by Formula III: 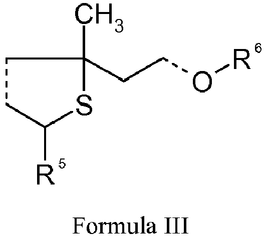
wherein R⁵ represents hydrogen, isopropyl or 1-hydroxy-1-methyl-ethyl; R⁶ represents hydrogen; the dotted line in the ring represents an optional carbon-carbon double bond; and the dotted line in the side chain represents an optional carbon-oxygen double bond,
with the proviso that, when the optional carbon-oxygen double bond is present, R⁶ is absent; and when R⁵ represents an isopropyl group and the optional carbon-carbon double bond is absent, the optional carbon-oxygen double bond is present and R⁶ is absent.

3. A compound represented by Formula VI: 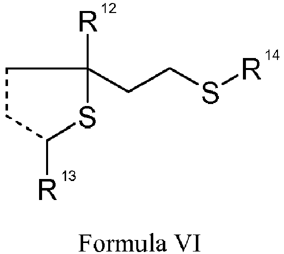 wherein
R¹² is a C₁-C₄ alkyl group;
R¹³ is selected from the group consisting of hydrogen, a C₁-C₄ alkyl group optionally containing a hydroxyl group and a C₁-C₄ alkenyl group optionally containing a hydroxyl group;
R¹⁴ is selected from the group consisting of hydrogen, a C₄-C₄ alkyl group and - C(O)R'⁵, wherein R¹⁵ is selected from the group consisting of hydrogen and a C₁-C₄ alkyl group; and
one of the dotted lines represents an optional carbon-carbon double bond.

4. The compound of claim 3 represented by Formula VIII: 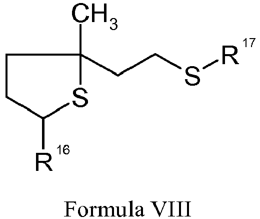
wherein R¹⁶ represents hydrogen, isopropyl or 1-hydroxy-1-methyl-ethyl; and
R¹⁷ is selected from the group consisting of a C₁-C₄ alkyl group and -C(O)R¹⁸,
wherein R¹⁸ is selected from the group consisting of hydrogen and a C₄-C₄ alkyl group.

5. A compound represented by Formula IX: 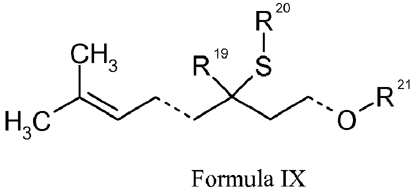 wherein
R¹⁹ is a C₁-C₄ alkyl group;
R²⁰ is selected from the group consisting of hydrogen and -C(O)R²², wherein R²² is selected from the group consisting of hydrogen and a C₁-C₄ alkyl group;
R²¹ is selected from the group consisting of hydrogen, a C₁-C₄ alkyl group and - C(O)R²³, wherein R²³ is selected from the group consisting of hydrogen and a C₁-C₄ alkyl group;
the dotted lines represent an optional carbon-carbon double bond and an optional carbon-oxygen double bond,
with the proviso that, when the optional carbon-oxygen double bond is present, R²¹ is absent.

6. The compound of claim 5 selected from the group consisting of:
3-mercapto-3,7-dimethyl-oct-6-en-1-ol;
3-mercapto-3,7-dimethyl-oct-6-enal;
S-[1-(2-hydroxy- ethyl)-1,5-dimethyl-hex-4-enyl]-thioacetate; and
3-acetylsulfanyl-3,7-dimethyl-oct-6-enyl acetate.

7. A flavor composition comprising an olfactory acceptable amount of the compound of any of the preceding claims.

8. The flavor composition of claim 7 further comprising a material selected from the group consisting of a foodstuff, a chewing gum, a dental product, an oral hygiene product and a medicinal product.

9. The flavor composition of claim 7, wherein the olfactory acceptable amount is from about 1 part per billion to about 1000 parts per million by weight; or
wherein the olfactory acceptable amount is from about 10 parts per billion to about 100 parts per million by weight; or
wherein the olfactory acceptable amount is from about 100 parts per billion to about 10 parts per million by weight.

10. A method of improving, enhancing or modifying a flavor composition through the addition of an olfactory acceptable amount of the compound of any of the preceding claims.

11. A fragrance composition comprising an olfactory acceptable amount of the compound of any of the preceding claims.

12. The fragrance composition of claim 11 further comprising a material selected from the group consisting of a polymer and a non-polymer, and optionally or preferably wherein the non-polymer is selected from the group consisting of an oligomer, a surfactant, an emulsifier, a fat, a wax, a phospholipid, an organic oil, a mineral oil, a petrolatum, a natural oil, a perfume fixative, a fiber, a starch, a sugar and a solid surface material.

13. The fragrance composition of claim 12, optional or preferable part, wherein the solid surface material is selected from the group consisting of zeolite and silica.

14. The fragrance composition of claim 11, wherein the olfactory acceptable amount is from about 0.005 to about 50 weight percent of the fragrance composition; or
wherein the olfactory acceptable amount is from about 0.5 to about 25 weight percent of the fragrance composition; or
wherein the olfactory acceptable amount is from about 1 to about 10 weight percent of the fragrance composition.

15. A method of improving, enhancing or modifying a fragrance composition through the addition of an olfactory acceptable amount of the compound of any of the preceding claims.

## Patentansprüche

1. Verbindung, dargestellt durch Formel I: 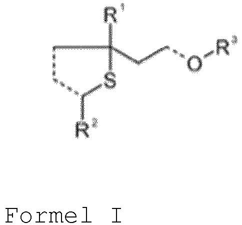 worin
R¹ eine C₁-C₄ Alkylgruppe ist;
R² ausgewählt ist aus der Gruppe bestehend aus Wasserstoff, einer C₁-C₄ Alkylgruppe, die wahlweise eine Hydroxylgruppe enthält, und einer C₁-C₄ Alkenylgruppe, die wahlweise eine Hydroxylgruppe enthält;
R³ ausgewählt ist aus der Gruppe bestehend aus Wasserstoff, einer C₁-C₄ Alkylgruppe und -C(O)R⁴, wobei R⁴ ausgewählt ist aus der Gruppe bestehend aus Wasserstoff und einer C₁-C₄ Alkylgruppe;
eine der gestrichelten Linien im Ring eine optionale Kohlenstoff-Kohlenstoff-Doppelbindung darstellt; und die gestrichelte Linie in der Seitenkette eine optionale Kohlenstoff-Sauerstoff-Doppelbindung darstellt,
mit der Maßgabe, dass, wenn die optionale Kohlenstoff-Sauerstoff-Doppelbindung vorhanden ist, R³ nicht vorhanden ist.

2. Verbindung nach Anspruch 1, dargestellt durch die Formel III: 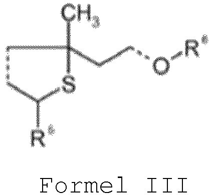
worin R⁵ Wasserstoff, Isopropyl oder 1-Hydroxy-1-methyl-ethyl darstellt; R⁶ Wasserstoff darstellt; die gestrichelte Linie im Ring eine optionale Kohlenstoff-Kohlenstoff-Doppelbindung darstellt; und die gestrichelte Linie in der Seitenkette eine optionale Kohlenstoff-Sauerstoff-Doppelbindung darstellt,
mit der Maßgabe, dass, wenn die optionale Kohlenstoff-Sauerstoff-Doppelbindung vorhanden ist, R⁶ abwesend ist; und wenn R⁵ eine Isopropylgruppe darstellt und die optionale Kohlenstoff-Kohlenstoff-Doppelbindung nicht vorhanden ist, die optionale Kohlenstoff-Sauerstoff-Doppelbindung vorhanden und R⁶ nicht vorhanden ist.

3. Verbindung, dargestellt durch Formel VI: 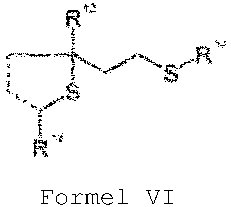 worin
R¹² eine C₁-C₄ Alkylgruppe ist;
R¹³ ausgewählt ist aus der Gruppe bestehend aus Wasserstoff, einer C₁-C₄ Alkylgruppe, die wahlweise eine Hydroxylgruppe enthält, und einer C₁-C₄ Alkenylgruppe, die wahlweise eine Hydroxylgruppe enthält;
R¹⁴ ausgewählt ist aus der Gruppe bestehend aus Wasserstoff, einer C₁-C₄ Alkylgruppe und -C(O)R¹⁵, wobei R¹⁵ ausgewählt ist aus der Gruppe bestehend aus Wasserstoff und einer C₁-C₄ Alkylgruppe; und
eine der gestrichelten Linien eine optionale Kohlenstoff-Kohlenstoff-Doppelbindung darstellt.

4. Verbindung nach Anspruch 3, dargestellt durch Formel VIII: 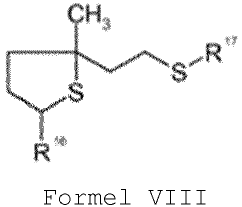
worin R¹⁶ Wasserstoff, Isopropyl oder 1-Hydroxy-1-methyl-ethyl darstellt; und
R¹⁷ ausgewählt ist aus der Gruppe bestehend aus einer C₁-C₄ Alkylgruppe und -C(O)R¹⁸,
wobei R¹⁸ ausgewählt ist aus der Gruppe bestehend aus Wasserstoff und einer C₁-C₄ Alkylgruppe.

5. Verbindung, dargestellt durch Formel IX: 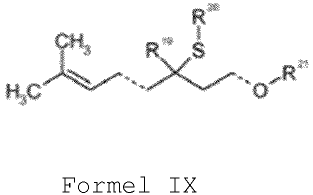 worin
R¹⁹ eine C₁-C₄ Alkylgruppe ist;
R²⁰ ausgewählt ist aus der Gruppe bestehend aus Wasserstoff und -C(O)R²², wobei R²² ausgewählt ist aus der Gruppe bestehend aus Wasserstoff und einer C₁-C₄ Alkylgruppe;
R²¹ ausgewählt ist aus der Gruppe bestehend aus Wasserstoff, einer C₁-C₄ Alkylgruppe und -C(O)R²³, wobei R²³ ausgewählt ist aus der Gruppe bestehend aus Wasserstoff und einer C₁-C₄ Alkylgruppe,
die gestrichelten Linien eine optionale Kohlenstoff-Kohlenstoff-Doppelbindung und eine optionale Kohlenstoff-Sauerstoff-Doppelbindung darstellen,
mit der Maßgabe, dass, wenn die optionale Kohlenstoff-Sauerstoff-Doppelbindung vorhanden ist, R²¹ nicht vorhanden ist.

6. Verbindung nach Anspruch 5, ausgewählt aus der Gruppe bestehend aus:
3-Mercapto-3,7-dimethyl-oct-6-en-1-ol;
3-Mercapto-3,7-dimethyl-oct-6-enal;
S-[1-(2-Hydroxyethyl)-1,5-dimethyl-hex-4-enyl]-thioacetat; und
3-Acetylsulfanyl-3,7-dimethyl-oct-6-enylacetat.

7. Geschmacksstoffzusammensetzung, die eine olfaktorisch annehmbare Menge der Verbindung nach einem der vorherigen Ansprüche umfasst.

8. Geschmacksstoffzusammensetzung nach Anspruch 7, die ferner ein Material umfasst, das ausgewählt ist aus der Gruppe, bestehend aus einem Nahrungsmittel, einem Kaugummi, einem Zahnprodukt, einem Mundhygieneprodukt und einem medizinischen Produkt.

9. Geschmacksstoffzusammensetzung nach Anspruch 7, wobei die olfaktorisch annehmbare Menge von etwa 1 pro Milliarde bis etwa 1000 pro Million Gewichtsteile beträgt; oder
wobei die olfaktorisch annehmbare Menge etwa 10 pro Milliarde bis etwa 100 pro Million Gewichtsteile beträgt;
oder worin die olfaktorisch annehmbare Menge etwa 100 pro Milliarde bis etwa 10 pro Million Gewichtsteile beträgt.

10. Verfahren zum Verbessern, Verstärken oder Modifizieren einer Geschmacksstoffzusammensetzung durch Zugabe einer olfaktorisch annehmbaren Menge der Verbindung nach einem der vorherigen Ansprüche.

11. Duftstoffzusammensetzung, die eine olfaktorisch annehmbare Menge der Verbindung nach einem der vorherigen Ansprüche umfasst.

12. Duftstoffzusammensetzung nach Anspruch 11, die ferner ein Material umfasst, das ausgewählt ist aus der Gruppe, bestehend aus einem Polymer und einem Nicht-Polymer, und wahlweise oder vorzugsweise wobei das Nicht-Polymer ausgewählt ist aus der Gruppe bestehend aus einem Oligomer, einem Tensid, einem Emulgator, einem Fett, einem Wachs, einem Phospholipid, einem organischen Öl, einem Mineralöl, einem Petrolatum, einem natürlichen Öl, einem Parfümfixativ, einer Faser, einer Stärke, einem Zucker und einem festen Oberflächenmaterial.

13. Duftstoffzusammensetzung nach Anspruch 12, wahlweiser oder bevorzugter Teil, wobei das feste Oberflächenmaterial ausgewählt ist aus der Gruppe bestehend aus Zeolith und Siliziumdioxid.

14. Duftstoffzusammensetzung nach Anspruch 11, wobei die olfaktorisch annehmbare Menge etwa 0,005 bis etwa 50 Gewichtsprozent der Duftstoffzusammensetzung beträgt; oder
wobei die olfaktorisch annehmbare Menge etwa 0,5 bis etwa 25 Gewichtsprozent der Duftstoffzusammensetzung beträgt; oder
wobei die olfaktorisch annehmbare Menge etwa 1 bis etwa 10 Gewichtsprozent der Duftstoffzusammensetzung beträgt.

15. Verfahren zum Verbessern, Verstärken oder Modifizieren einer Duftstoffzusammensetzung durch Zugabe einer olfaktorisch annehmbaren Menge der Verbindung nach einem der vorherigen Ansprüche.

## Revendications

1. Composé représenté par la Formule I : 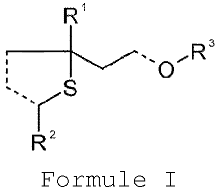 dans lequel
R¹ est un groupement C₁-C₄-alkyle ;
R² est choisi dans le groupe constitué par hydrogène, un groupement C₁-C₄-alkyle contenant éventuellement un groupement hydroxyle et un groupement C₁-C₄-alcényle contenant éventuellement un groupement hydroxyle ;
R³ est choisi dans le groupe constitué par hydrogène, un groupement C₁-C₄-alkyle et -C(O)R⁴, où R⁴ est choisi dans le groupe constitué par hydrogène et un groupement C₁-C₄-alkyle ;
l'une des lignes en pointillés dans le cycle représente une double liaison carbone-carbone facultative ; et la ligne en pointillés dans la chaîne latérale représente une double liaison carbone-oxygène facultative ;
à condition que, lorsque la double liaison carbone-oxygène facultative est présente, R³ soit absent.

2. Composé selon la revendication 1, représenté par la Formule III : 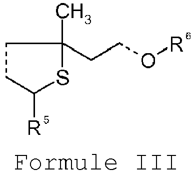 dans lequel
R⁵ représente hydrogène, isopropyle ou 1-hydroxy-1-méthyléthyle ; R⁶ représente hydrogène ; la ligne en pointillés dans le cycle représente une double liaison carbone-carbone facultative ; et la ligne en pointillés dans la chaîne latérale représente une double liaison carbone-oxygène facultative ;
à condition que, lorsque la double liaison carbone-oxygène facultative est présente, R⁶ soit absent ; et lorsque R⁵ représente un groupement isopropyle et la double liaison carbone-carbone facultative est absente, la double liaison carbone-oxygène facultative soit présente et R⁶ soit absent.

3. Composé représenté par la Formule VI : 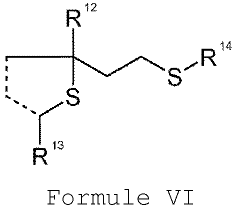 dans lequel
R¹² est un groupement C₁-C₄-alkyle ;
R¹³ est choisi dans le groupe constitué par hydrogène, un groupement C₁-C₄-alkyle contenant éventuellement un groupement hydroxyle et un groupement C₁-C₄-alcényle contenant éventuellement un groupement hydroxyle ;
R¹⁴ est choisi dans le groupe constitué par hydrogène, un groupement C₁-C₄-alkyle et -C(O)R¹⁵, où R¹⁵ est choisi dans le groupe constitué par hydrogène et un groupement C₁-C₄-alkyle ; et
l'une des lignes en pointillés représente une double liaison carbone-carbone facultative.

4. Composé selon la revendication 3, représenté par la Formule VIII : 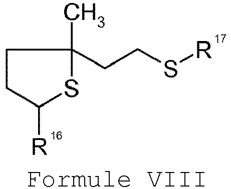 dans lequel
R¹⁶ représente hydrogène, isopropyle ou 1-hydroxy-1-méthyléthyle ; et
R¹⁷ est choisi dans le groupe constitué par un groupement C₁-C₄-alkyle et -C(O)R¹⁸,
où R¹⁸ est choisi dans le groupe constitué par hydrogène et un groupement C₁-C₄-alkyle.

5. Composé représenté par la Formule IX : 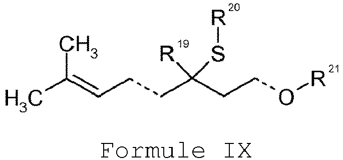 dans lequel
R¹⁹ est un groupement C₁-C₄-alkyle ;
R²⁰ est choisi dans le groupe constitué par hydrogène et -C(O)R²² , où R²² est choisi dans le groupe constitué par hydrogène et un groupement C₁-C₄-alkyle ;
R²¹ est choisi dans le groupe constitué par hydrogène, un groupement C₁-C₄-alkyle et -C(O)R²³, où R²³ est choisi dans le groupe constitué par hydrogène et un groupement C₁-C₄-alkyle ;
les lignes en pointillés représentent une double liaison carbone-carbone facultative et une double liaison carbone-oxygène facultative,
à condition que, lorsque la double liaison carbone-oxygène facultative est présente, R²¹ soit absent.

6. Composé selon la revendication 5, choisi dans le groupe constitué par :
le 3-mercapto-3,7-diméthyloct-6-én-1-ol ;
le 3-mercapto-3,7-diméthyloct-6-énal ;
le thioacétate de S-[1-(2-hydroxyéthyl)-1,5-diméthylhex-4-ényl] ; et
l'acétate de 3-acétylsulfanyl-3,7-diméthyloct-6-ényle.

7. Composition d'arôme, comprenant une quantité acceptable olfactive du composé selon l'une quelconque des revendications précédentes.

8. Composition d'arôme selon la revendication 7, comprenant en outre un matériau choisi dans le groupe constitué par un produit alimentaire, un chewing-gum, un produit dentaire, un produit d'hygiène orale et un produit médicinal.

9. Composition d'arôme selon la revendication 7, dans laquelle la quantité acceptable olfactive va d'environ 1 partie par milliard à environ 1000 parties par million en poids ; ou
où la quantité acceptable olfactive va d'environ 10 parties par milliard à environ 100 parties par million en poids ;
où la quantité acceptable olfactive va d'environ 100 parties par milliard à environ 10 parties par million en poids.

10. Méthode destinée à améliorer, augmenter ou modifier une composition d'arôme par l'intermédiaire de l'addition d'une quantité acceptable olfactive du composé selon l'une quelconque des revendications précédentes.

11. Composition de parfum comprenant une quantité acceptable olfactive du composé selon l'une quelconque des revendications précédentes.

12. Composition de parfum selon la revendication 11, comprenant en outre un matériau choisi dans le groupe constitué par un polymère et un non polymère, et éventuellement ou préférablement où le non polymère est choisi dans le groupe constitué par un oligomère, un agent tensioactif, un émulsifiant, une matière grasse, une cire, un phospholipide, une huile organique, une huile minérale, un pétrolatum, une huile naturelle, un fixateur de parfum, une fibre, un amidon, un sucre, et un matériau de surface solide.

13. Composition de parfum selon la revendication 12, partie facultative ou préférable, dans laquelle le matériau de surface solide est choisi dans le groupe constitué par la zéolithe et la silice.

14. Composition de parfum selon la revendication 11, dans laquelle la quantité acceptable olfactive constitue d'environ 0,005 à environ 50% en poids de la composition de parfum ; ou
où la quantité acceptable olfactive constitue d'environ 0,5 à environ 25% en poids de la composition de parfum ; ou
où la quantité acceptable olfactive constitue d'environ 1 à environ 10% en poids de la composition de parfum.

15. Méthode destinée à améliorer, augmenter ou modifier une composition de parfum par l'intermédiaire de l'addition d'une quantité acceptable olfactive du composé selon l'une quelconque des revendications précédentes.
